# EUROPÄISCHE PATENTSCHRIFT

(11) **EP 1 554 259 B1**
(45) Veröffentlichungstag und Bekanntmachung des Hinweises auf die Patenterteilung: **27.12.2006**
(21) Anmeldenummer: 03808700.3
(22) Anmeldetag: 27.09.2003
(51) Int. Cl.: C07D 249/18, C07D 401/06, C07D 403/06, A61K 31/4192, A61P 3/10

(54) **NEUE BICYCLISCHE INHIBITOREN DER HORMON SENSITIVEN LIPASE**
NOVEL BICYCLIC INHIBITORS OF HORMONE SENSITIVE LIPASE
INHIBITEURS BICYCLIQUES DE LA LIPASE HORMONO-SENSIBLE

(30) Priorität: 12.10.2002 DE 10247680
(43) Veröffentlichungstag der Anmeldung: 20.07.2005
(73) Patentinhaber: Sanofi-Aventis Deutschland GmbH, 65929 Frankfurt am Main (DE)
(72) Erfinder: PETRY, Stefan, 65929 Frankfurt (DE); BARINGHAUS, Karl-Heinz, 61200 Wölfersheim (DE); TENNAGELS, Norbert, 65931 Frankfurt (DE); MUELLER, Guenter, 65843 Sulzbach (DE); HEUER, Hubert, 55270 Schwabenheim (DE)
(86) Internationale Anmeldenummer: PCT/EP2003/010765
(87) Internationale Veröffentlichungsnummer: WO 2004/035550

(56) Entgegenhaltungen:
- WO-A-01/17981
- WO-A-02/090355
- WO-A-03/051842
- J. Chem. Research (S) 1999, 230-231

## Beschreibung

Benzotriazole sind bereits aus den verschiedensten Gebieten bekannt, wie z.B. der Fotochemie (US 4,255,510, Kodak) oder als Orexin Antagonisten (WO 02/090355, SKB). Ferner ist die Synthese zur Herstellung von Benzotriazolen von Katritzky et al. in J. Org. Chem. 1997, 62, 4155-4158 und in J.Chem. Research (s) 1999, 230-231 beschrieben. Bekannt sind ferner Carbamate als Lipase-Inhibitoren wie z.B. Shamkant Patkar et al. in Paul Woolley, Steffen B. Petterson (ed), Lipase (1994) 207-227 oder WO 03/051842.

Überraschenderweise konnte nun gezeigt werden, dass die erfindungsgemäßen Benzotriazole Wirkung an der HSL, der hormon sensitiven Lipase zeigen.

Die Erfindung betritt Benzotriazole der allgemeinen Formel I, 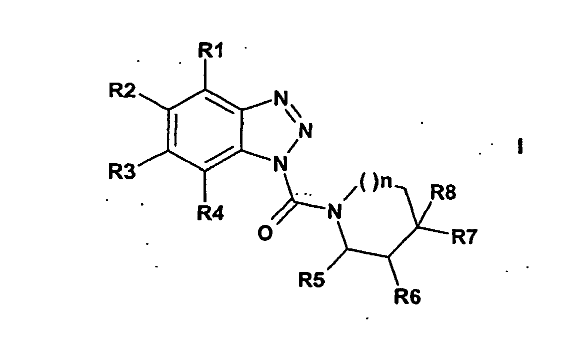 worin bedeuten :
- R1 bis R8: H,
wobei einer dieser Reste R2 oder R3 stehen kann für:
Br, Cl, CH₃, CN, NH₂, NO₂, CF₃, OCH₃, Phenoxy, Benzoyl, CH(OH)-Phenyl, S-Cyclohexyl, CO-OCH₃;
oder
zwei Substituenten aus dieser Reihe sind:
R1 = Cl und R3 = CF₃ oder
R2=Fund R3=Cl;
- n: eine ganze Zahl von 0, 1 oder 2; und
einer der Substituenten R6 oder R7 stehen kann für:
- R6: CH₃;
- R7: CH₃, C₂H₅; CH(CH₃)₂, C(CH₃)₃, CF₃, Br, Cl, Benzyl oder CO- OC₂H₅; oder
R6 und R7 beide CH₃ sind; oder
der Ring anstelle R6 und R7 eine Doppelbindung enthalten kann oder
- R5 und R6 oder R6 und R7: zusammen mit den sie tragenden C-Atomen für einen benzannellierten Ring oder falls n = 0 ist, auch für Cyclohexandiyl stehen kann, wobei im Falle des Ringschlusses R6/R7 dieser Substituent gegebenenfalls einfach durch NH₂ oder NO₂ oder einbis zweifach durch OCH₃ substituiert sein kann; und
R7 und R8 zusammen Cyclopentyl, Diazirin oder =CH₂;
wobei die Verbindungen mit R1 bis R5 und R8 =H, n = 1 und R6/R7 = benzannelliert und R1, R3-R8 = H , R2 = CH₃ und n = 1 ausgenommen sind.

Die Erfindung bezieht sich auf Verbindungen der Formel I, in Form ihrer Racemate, racemischen Mischungen und reinen Enantiomere sowie auf ihre Diastereomere und Mischungen davon.

Die Alkylreste können sowohl geradkettig wie verzweigt sein. Halogen steht für Fluor, Chlor oder Brom, besonders für Fluor oder Chlor.

Bevorzugt sind Benzotriazole der Formel I,
worin bedeuten :
- R1 bis R8: H;
wobei einer dieser Reste R2 oder R3 stehen kann für:
- R2: Br, Cl, CN, NO₂, CF₃, OCH₃, Phenoxy, Benzoyl, CH(OH)-Phenyl, S-Cyclohexyl, CO-OCH₃;
- R3: CH₃, CN, Br, Cl, NH₂, NO₂, Benzoyl.

Besonders bevorzugt sind die Benzotriazole der allgemeinen Formel I, worin bedeuten:
- R1 bis R8: H;
wobei einer dieser Reste R2 oder R3 stehen kann für:
- R2: Br, Cl, NO₂, OCH₃, Phenoxy, CO-OCH₃;
- R3: NH₂; oder
zwei Substituenten aus dieser Reihe sind:
R2 = F und R3 = Cl;
- n: eine ganze Zahl von 1 oder 2 und
einer der Substituenten R5 oder R6 stehen kann für :
- R6: CH₃;
- R7: CH₃, CF₃ oder Br; oder
der Ring anstelle R6 und R7 eine Doppelbindung enthalten kann oder
- R6 und R7: zusammen mit den sie tragenden C-Atomen für einen benzannellierten Ring stehen kann, der gegebenenfalls einfach durch NH₂ oder einbis zweifach durch OCH₃ substituiert sein kann; und
R7 und R8 zusammen Cyclopentyl oder
- n: eine ganze Zahl von 0; und
R6 und R7 zusammen mit den sie tragenden C-Atomen für einen benzannellierten Ring oder Cyclohexandiyl stehen kann; oder

Benzotriazole der allgemeinen Formel I, worin bedeuten
- R1 bis R8: H;
0 wobei einer dieser Reste R2 oder R3 stehen kann für:
- R2: Br, CN, CF₃, OCH₃, Phenoxy, Benzoyl, CH(OH)-Phenyl, S-Cyclohexyl;
- R3: CN, Br, Cl, NO₂, Benzoyl; oder
zwei Substituenten aus dieser Reihe sind:
R1 = Cl und R3 = CF₃;
- n: eine ganze Zahl von 1; und
einer der Substituenten R6 oder R7 stehen kann für:
- R6: CH₃;
- R7: CH₃, C₂H₅; CH(CH₃)₂, C(CH₃)₃, Benzyl oder CO-OC₂H₅; oder
R6 und R7 beide CH₃ sind; oder
der Ring anstelle R6 und R7 eine Doppelbindung enthalten kann oder
- R5 und R6 oder R6 und R7: zusammen mit den sie tragenden C-Atomen für einen benzannellierten Ring stehen kann;
wobei die Verbindungen mit R1 bis R5 und R8 =H, n = 1 und R6/R7 = benzannelliert ausgenommen ist.

Ganz besonders bevorzugt sind die Benzotriazole der folgendenStrukturen: 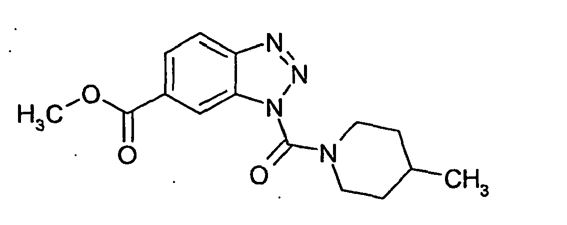 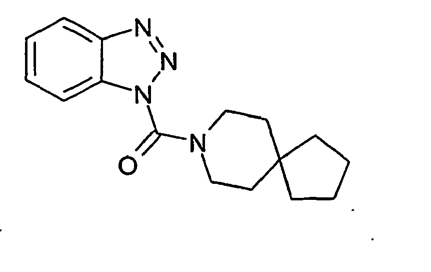 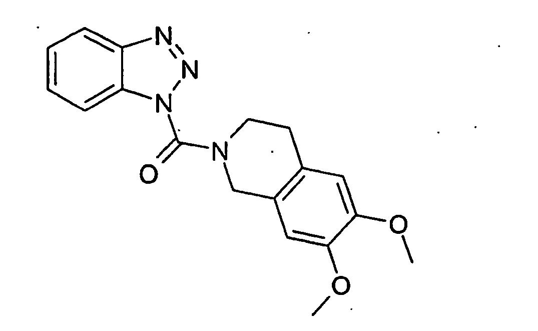 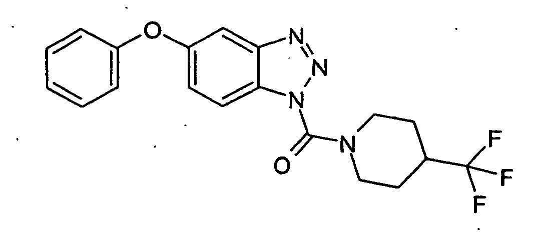 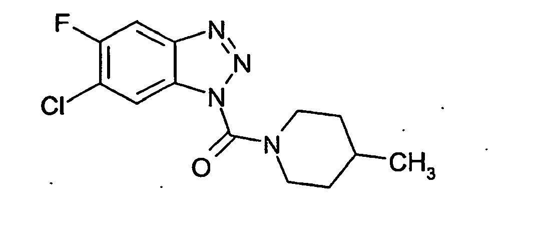 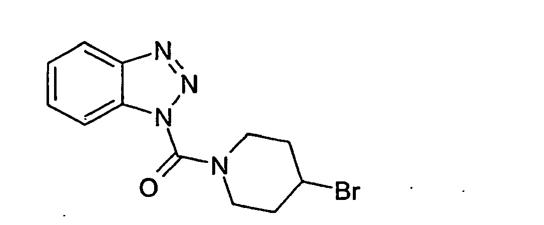 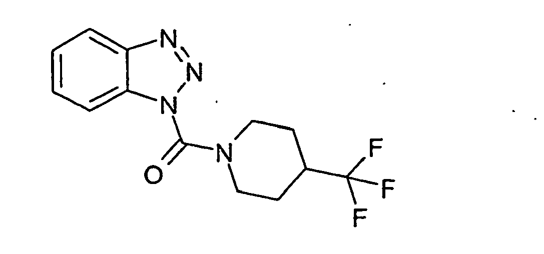 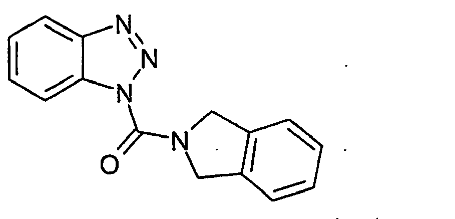 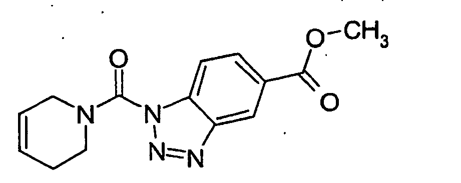 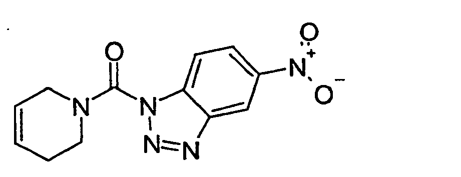 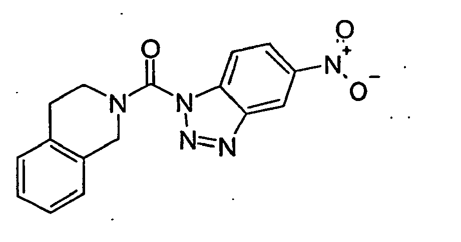 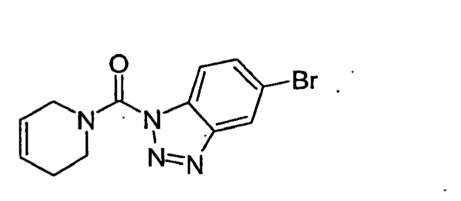 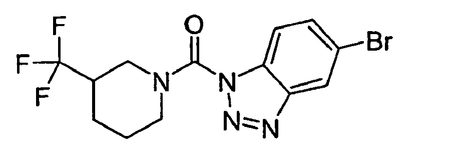 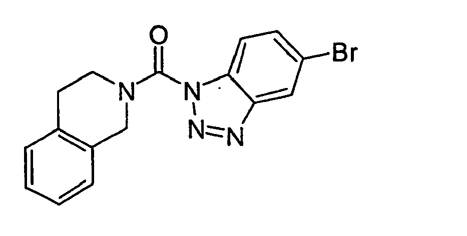 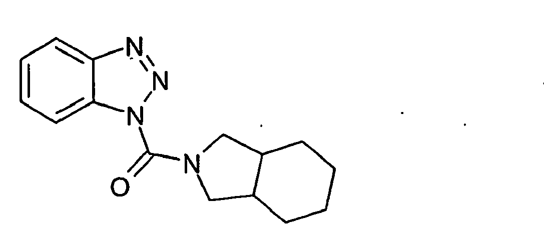 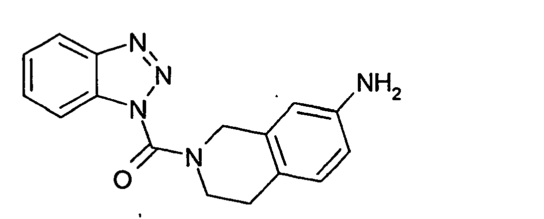 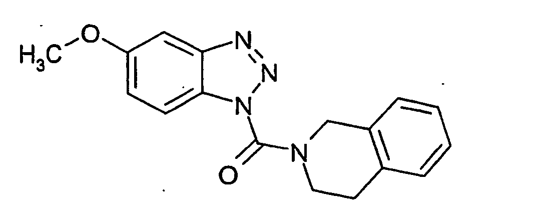 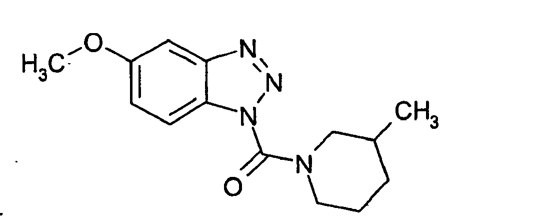 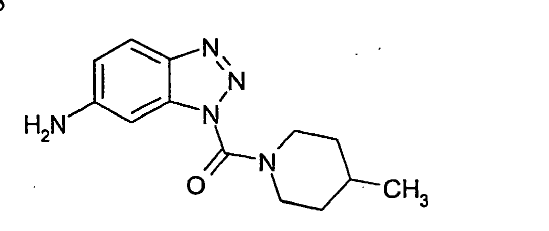 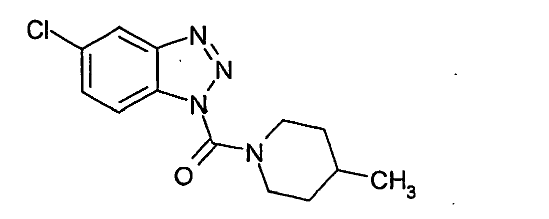 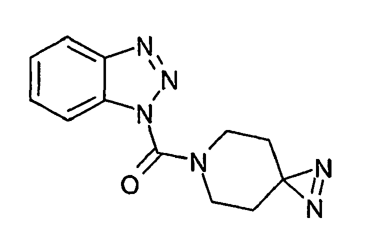 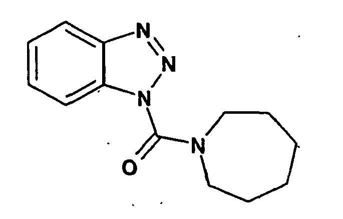 oder die Benzotriazole der folgenden Sturkturen:

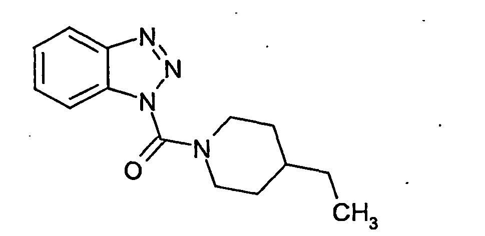

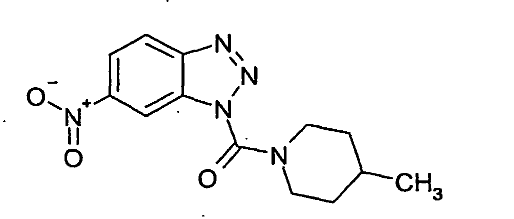

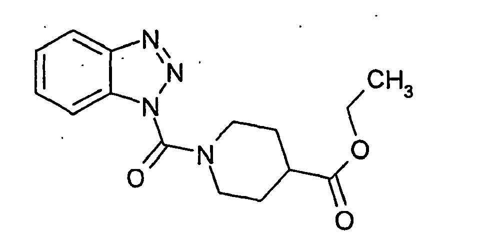

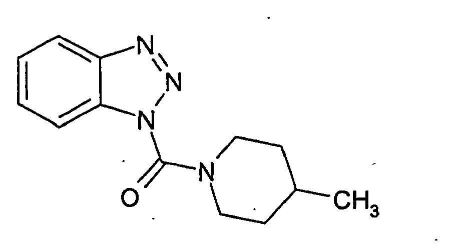

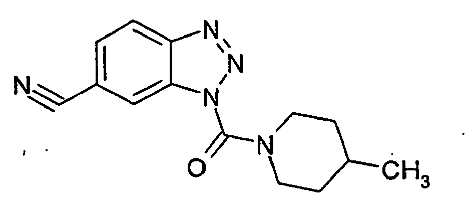

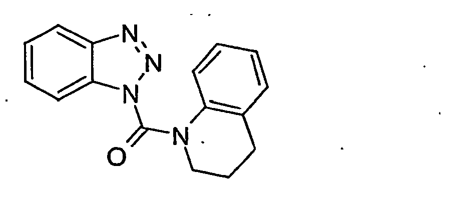

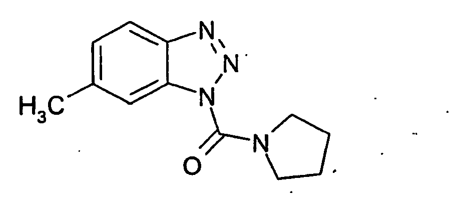

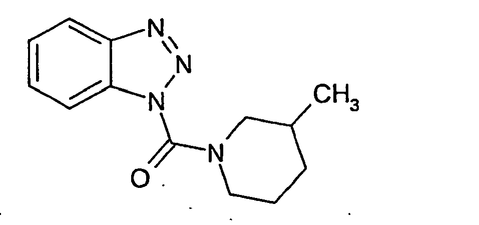

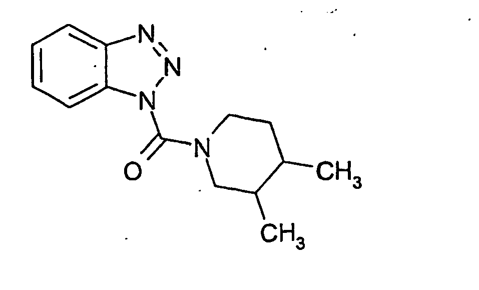

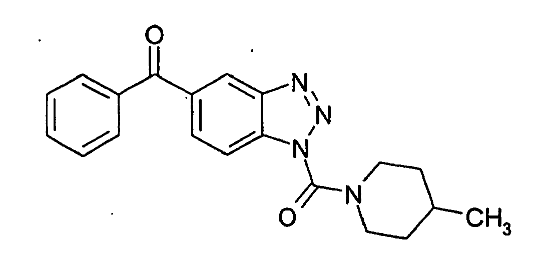

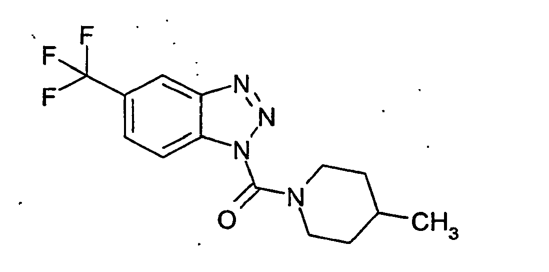

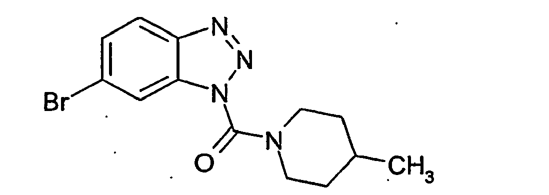

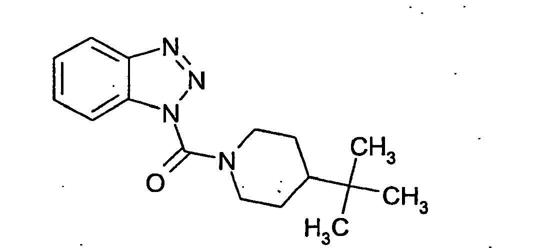

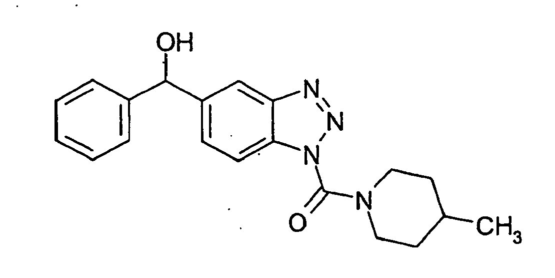

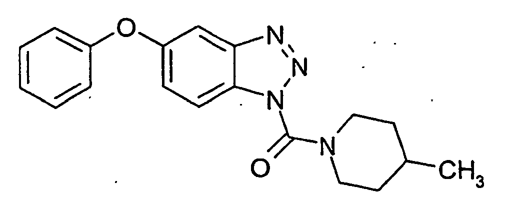

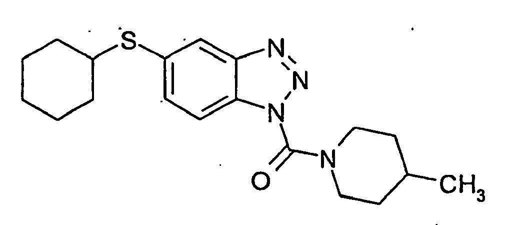

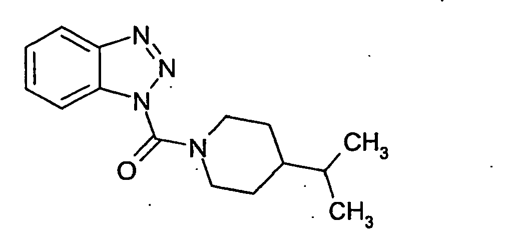

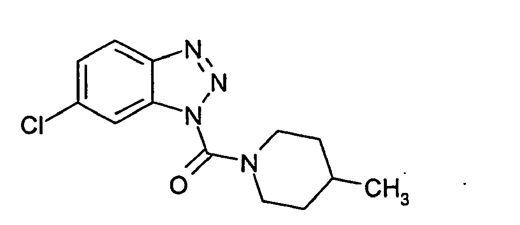

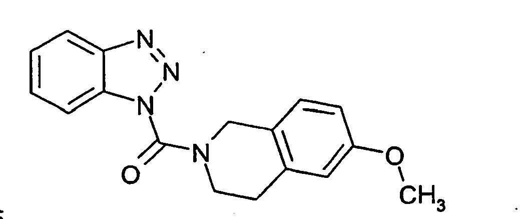

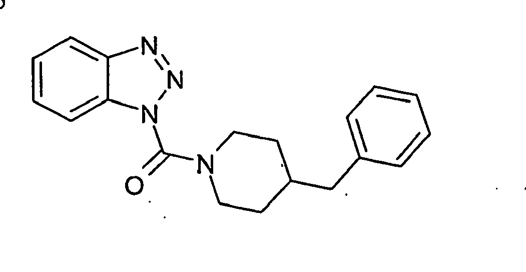

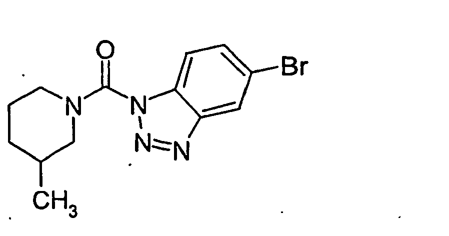

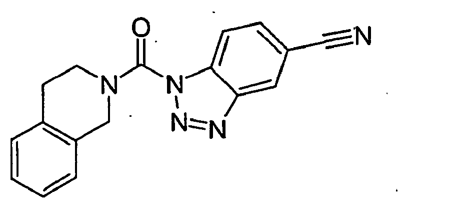

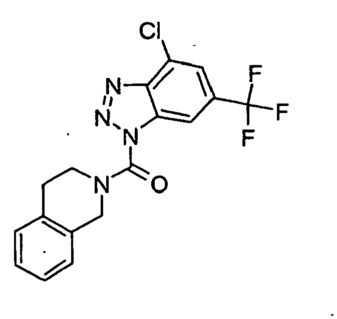

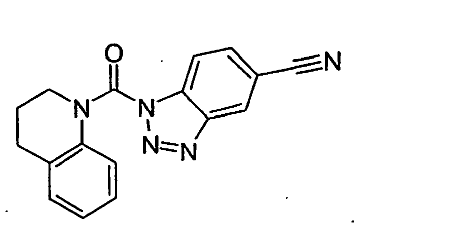

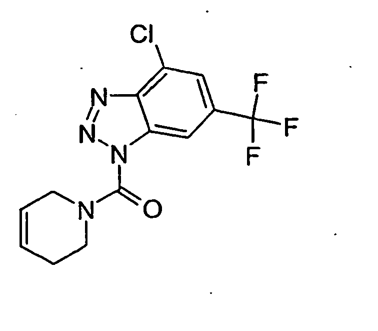

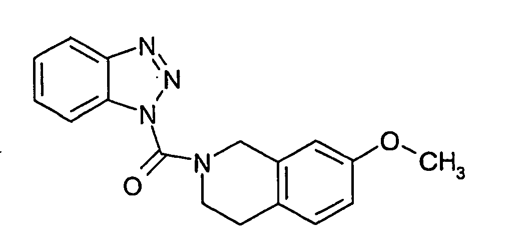

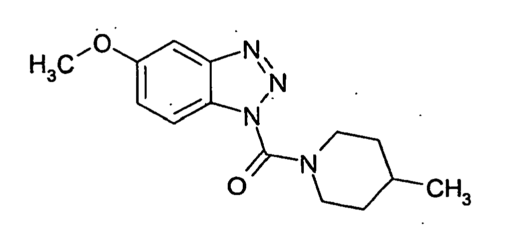

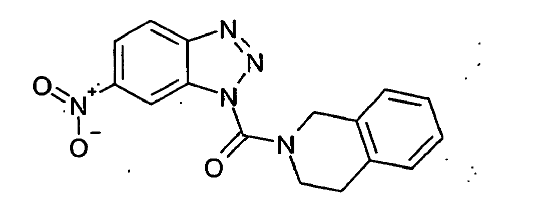

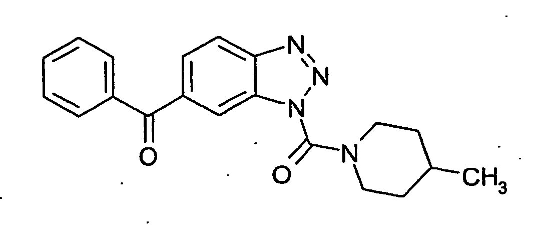

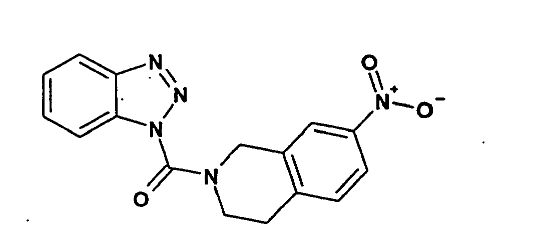

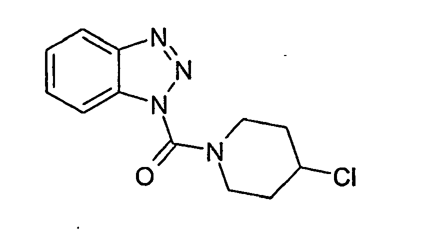

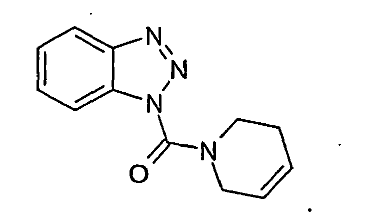

Ferner sind ganz besonders bevorzugt Benzotriazole der folgenden Strukturen: 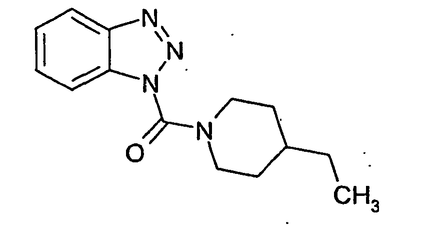 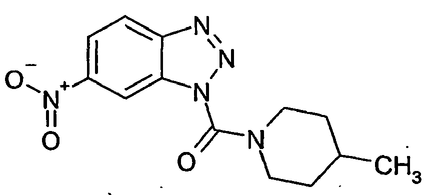 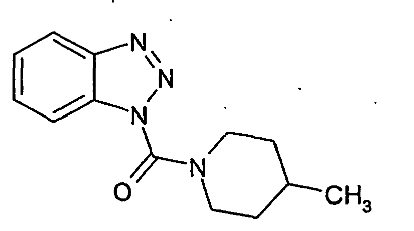 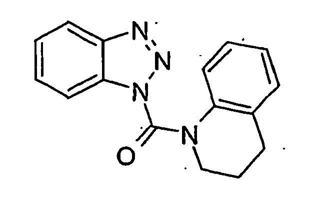 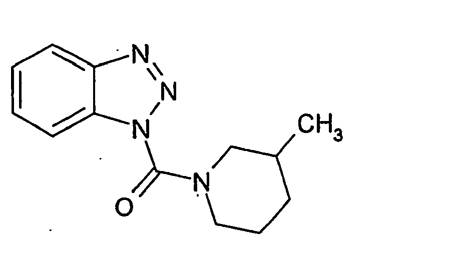 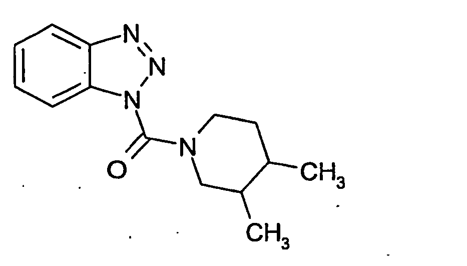 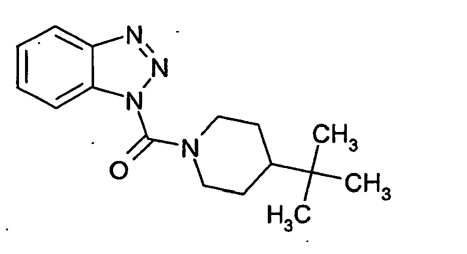 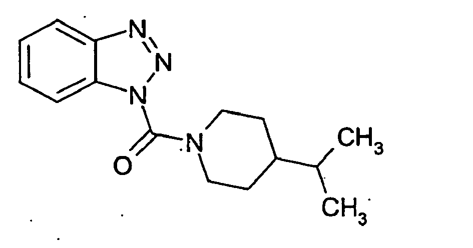 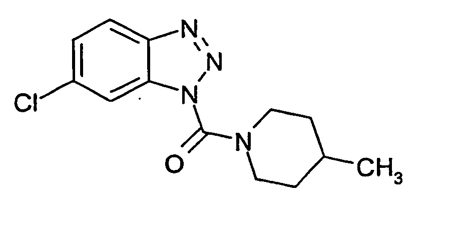 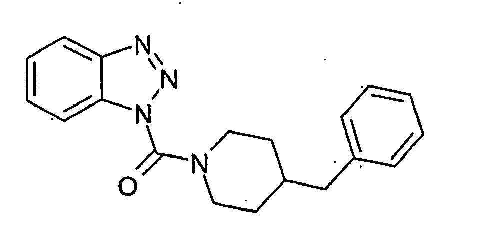 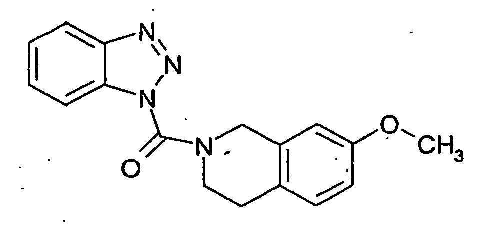 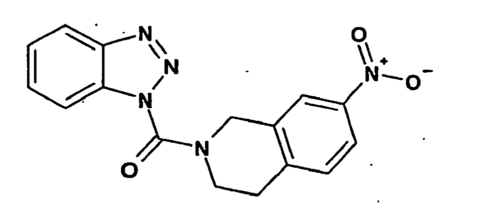 und die Benzotriazole der folgenden Strukturen:

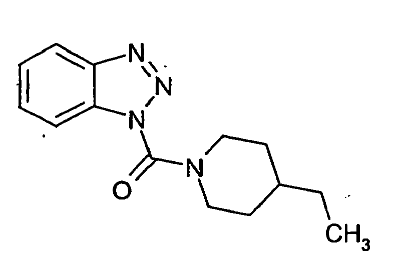

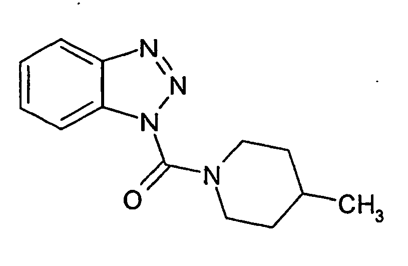

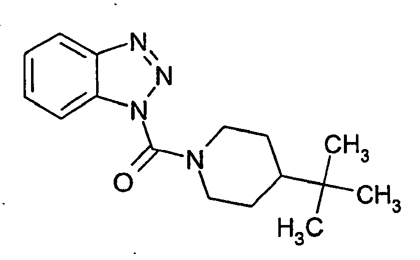

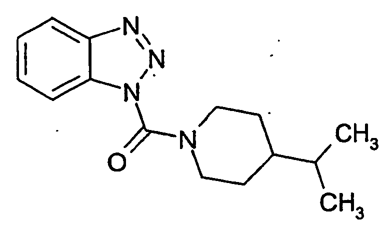

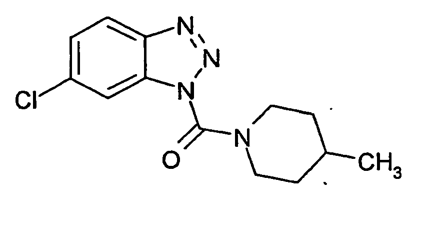

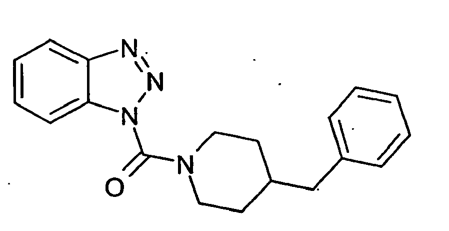

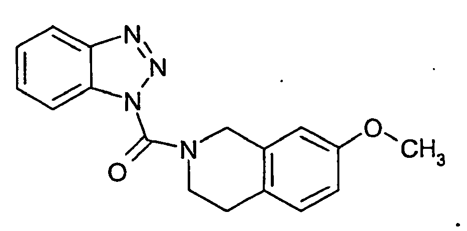

Pharmazeutisch verträgliche Salze sind aufgrund ihrer höheren Wasseriöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze, Solvate und physiologisch funktionellen Derivate wie hierin beschrieben.

Die Verbindung(en) der Formel (I) können auch in Kombination mit weiteren Wirkstoffen verabreicht werden.

Die Menge einer Verbindung gemäß Formel 1, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z. B. sublinguale) und parenterale (z. B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1 % bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

### Als weitere Wirkstoffe für die Kombinationspräparate sind geeignet:

Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe. Die oral wirksamen hypoglykämischen Wirkstoffe umfassen vorzugsweise Sulphonylfharnstoffe, Biguanidine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/262.65 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in WO 00/64888, WO 00/64876, WO 03/020269 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht.

Bei einer Ausführungsform der Erfindung werden die Verbindungen der Formel in Kombination mit Gallensäureresorptionsinhibitor (siehe z.B. US 6,245,744 oder US 6,221,897), wie z.B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. Cl-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit mehr als einer der vorstehend genannten Verbindungen, z.B. in Kombination mit - einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) , Orexin-Antagonisten (z.B. 1-(2-Methyl-benzoxazol-6-yl)-3-[1,5]naphthyridin-4-yl-harnstoff; hydrochlorid (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00 /63208)); TNF-Agonisten, CRF-Antagonisten (z.B. [2-Methyl-9-(2,4,6-trimethyl-phenyl)-9H-1 ,3,9-triaza-fluoren-4-yl]-dipropyl-amin (WO 00/66585)), CRF BP-Antagonisten (z.B. Urocortin), Urocortin-Agonisten,β3-Agonisten (z.B. 1-(4-Chloro-3-methanesulfonylmethyl-phényl)-2-[2-(2,3-dimethyl-1H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-acetic acid Trifluoressigsäuresalz (WO 99/15525)); Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B: Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin.
Bei einer Ausführungsform ist der weitere Wirkstoff Fenfluramin oder Dexfenfluramin.
Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

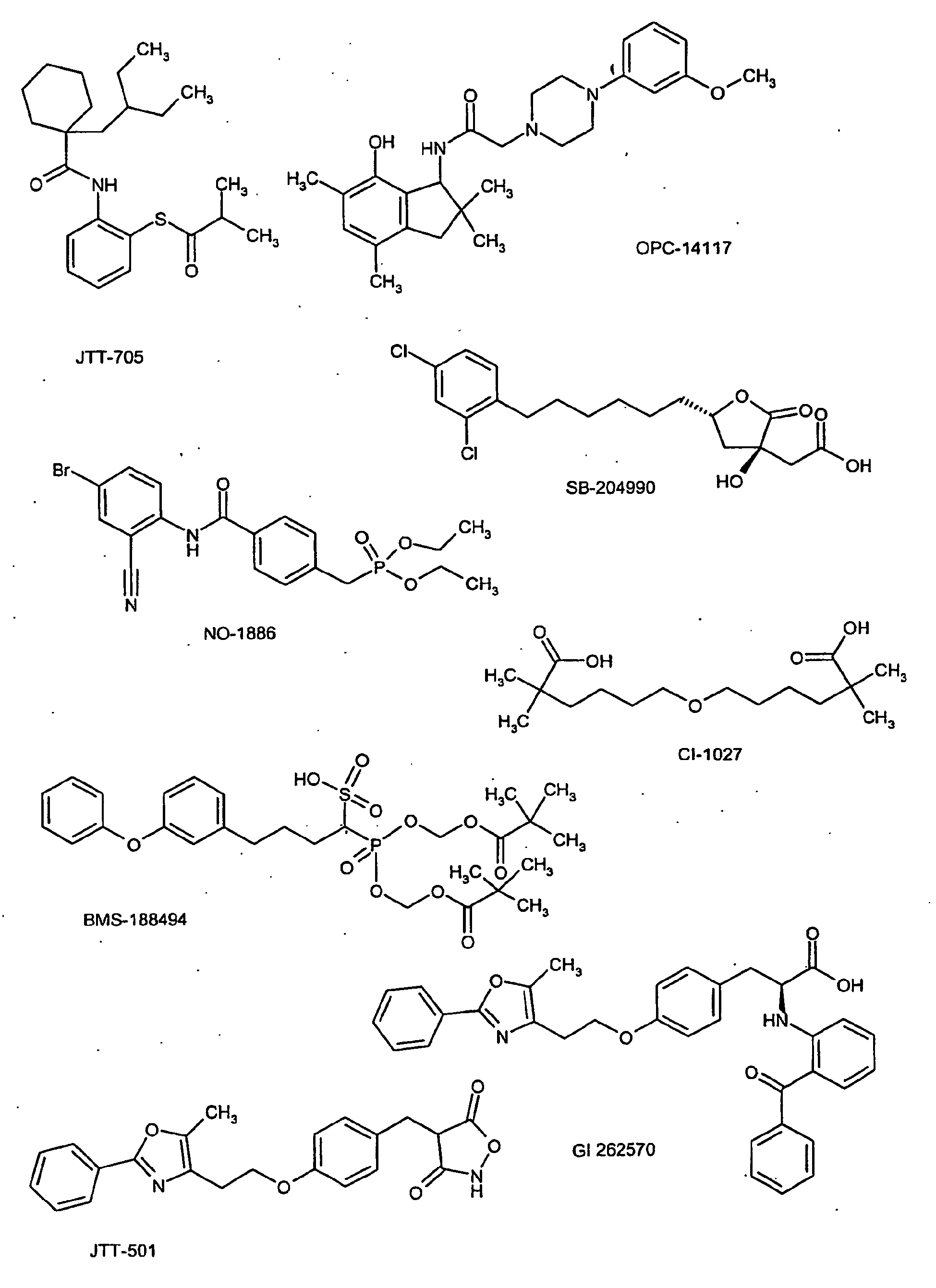

Die Herstellung der erfindungsgemäßen Benzotriazole der allgemeinen Formel I erfolgt nach an und für sich bekannten Methoden z.B. durch Acylierung von substituierten, bzw. unsubstituiertem Benzotriazol 2 mit Carbamoylchloriden 3 (Methode A), oder in zwei Stufen durch Umsetzung von Benzotriazolen mit Phosgen und weiterer Umsetzung des erhaltenen Benzotriazolcarbonsäurechlorids mit Aminen (Methode B).

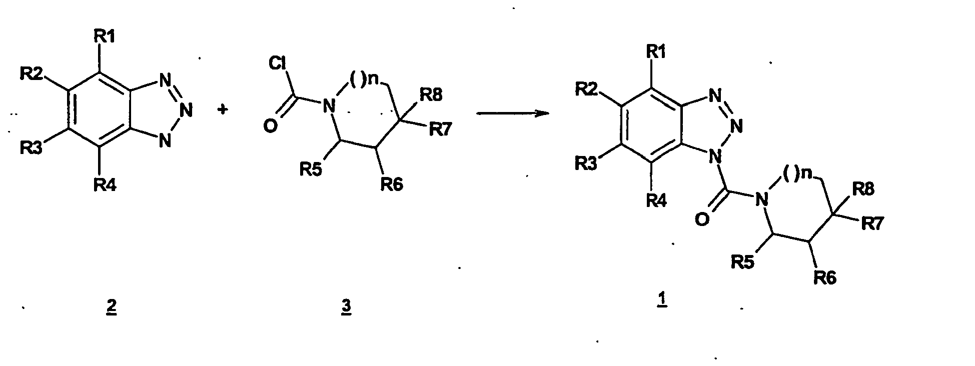

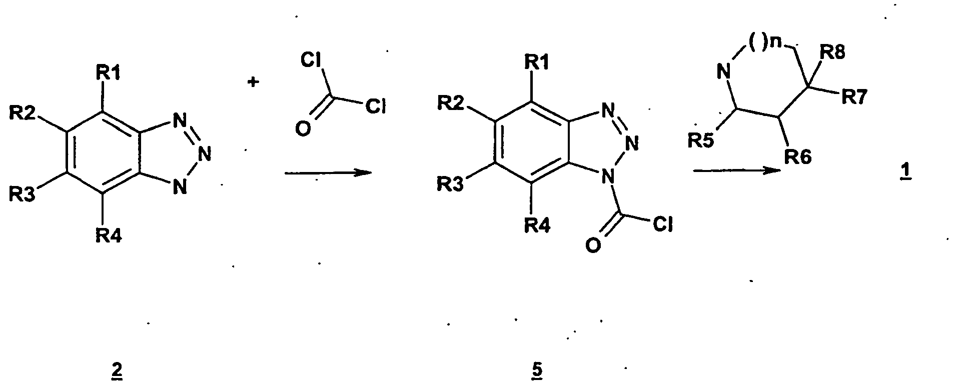

Da bei diesen Reaktionen in der Regel Säuren freigesetzt werden, empfielt es sich zur Beschleunigung Basen wie Pyridin, Triethylamin, Natronlauge oder Alkalicarbonate zuzusetzen. Die Reaktionen können in weiten Temperaturbereichen durchgeführt werden. In der Regel hat es sich als vorteilhaft herausgestellt, bei 0°C bis zum Siedepunkt des verwendeten Lösungsmittels zu arbeiten. Als Lösemittel kommen beispielsweise Methylenchlorid, THF, DMF, Toluol, Essigester, nHeptan, Dioxan, Diethylether zum Einsatz.

Die erfindungsgemäßen Verbindungen der allgemeinen Formel I besitzen eine überraschende hemmende Wirkung an der hormonsensitiven Lipase, HSL, einem allosterischen Enzym in Adipozyten, das durch Insulin gehemmt wird und für den Abbau von Fetten in Fettzellen und damit für die Überführung von Fettbestandteilen in die Blutbahn verantwortlich ist. Eine Hemmung dieses Enzyms entspricht also einer insulinähnlichen Wirkung der erfindungsgemäßen Verbindungen, die letztlich zu einer Verminderung von freien Fettsäuren im Blut und von Blutzucker führt. Sie können also eingesetzt werden bei Entgleisungen des Stoffwechsels wie zum Beispiel beim nicht insulinabhängigen Diabetes Mellitus, beim diabetischen Syndrom, beim Syndrom X und bei direkter Schädigung des Pankreas.
Eine Hemmung der HSL in β-Zellen sollte zu einer direkten Erholung der Freisetzung von Insulin führen (M. Winzell et al., Diabetes, Vol 52, August 2003, 2057-2065). Die erfindungsgemäßen Verbindungen der Formel I können daher auch zur Insulin-Freisetzung dienen.

Die Wirkung der erfindungsgemäßen Verbindungen der Formel wurde an folgendem Enzymtestsytem geprüft:

### Substratpräparation:

### Präparation des NAG (NBD-Monoacylglycerid) Substrats:

6 mg Phosphatidylcholin und 6 mg Phosphatidylinositol werden in je 1 ml Chloroform gelöst. 10 mg NAG werden in 1 ml Chlorofom gelöst. Zwei Teile Phosphatidylinositollösung (z.B. 83.5 µl) und ein Teil Phosphatidylcholinlösung (z.B. 41.5 µl) und 100 µl NAG Lösung werden in Plastikszintillationsgefäßen zusammenpipettiert (Endkonzentration im Test: 0.0375 mg Phospholipid/ml; 0.05 mg/ NAG/ml). Das Chloroform (225 µl Gesamtvolumen) wird durch Überblasen mit einem N2-Strom komplett entfernt. Das getrocknete Substrat kann für bis zu 3 Tagen bei 4°C aufbewahrt werden. Zur Herstellung der Phospholipidvesikel/Micellen mit interkaliertem NAG (am Testtag) wird das getrocknete Substrat in 20 ml Assaypuffer (25 mM Tris/HCl, pH 7.4; 150 mM NaCl) aufgenommen und zwei Ultraschallbehandlungen mit einem Ultraschallstab (Branson Sonifier Type II, Standardmikrospitze): 1. Behandlung Einstellung 2, 2 x 1 min, dazwischen jeweils 1 min auf Eis; 2. Behandlung Einstellung 4, 2 x 1 min, dazwischen jeweils 1 min auf Eis. Während dieser Prozedur verändert sich sich die Farbe der Substratlösung von gelb (Extinktionsmaximum 481 nm) nach rot (Extinktionsmaximum 550 nm) durch Interkalation von NAG zwischen die Phospholipidmoleküle der Vesikel/Micellen. Vor Benutzung als Substrat (innerhalb der nächsten 2 h) wird die Lösung noch 15 min auf Eis inkubiert.

### Indirekter NAG Assay:

Der Assay wird in 1.5-ml Eppendorfgefäßen oder 96-Lochplatten für 60 min bei 30°C durchgeführt. Für das Auffinden von Inhibitoren der HSL werden 10 µl der Testsubstanz in Assaypuffer (25 mM Tris/HCl, pH 7.4; 150 mM NaCl) in Gegenwart von 16.6 % DMSO vorgelegt. 180 µl der Substratlösung (20 µg/ml Phosphatidylcholin, 10 µg/ml Phosphatidylinositol, 50 µg/ml NAG in Assaypuffer) werden hinzugegeben: Nach einer Vorinkubation für 15 min bei 30°C werden 20 µl der Enzymlösung in Assaypuffer (1- bis 4-fach verdünnt hinzupipettiert und die Extinktion bei 480 nm in einem Küvettenphotometer (0.5 ml-Küvette) bzw. Mikrotiterplattenlesegerät sofort gemessen. Nach 60 min Inkubation bei 30°C wird die Extinktion erneut gemessen. Die Zunahme der Extinktion bei 480 nm ist ein Maß für die Enzymaktivität. Unter Standardbedingungen führen 20 µg partiell gereinigte HSL zu einer Extinktionsänderung von 0.4 = 4000 arb. units.

### Direkter NAG Assay:

Alternativ zur Messung der Extinktionsänderung der Substratlösung werden die Produkte der HSL Reaktion durch Phasentrennung/Dünnschichtchromatographie untersucht. Dazu wird der Inkubationsansatz (200 µl Gesamtvolumen, s. indirekter NAG Assay) in 2 ml Eppendorfgefäßeh mit 1.3 ml Methanol/Chloroform/Heptan (10:9:7) und anschließend mit 0.4 ml 0.1 M NaOH versetzt. Nach intensivem Mischen (10 sec) wird die Phasentrennung durch Zentrifugation (800xg, 20 min, Raumtemperatur) eingeleitet. Von der wässrigen oberen Phase werden äquivalente Volumen (z. B. 0.4 ml) abgenommen und die Extinktion photometrisch bei 481 nm bestimmt. Zur Dünnschichtchromatographie wird die wässrige Phase getrocknet (SpeedVac) und dann in 50 µl Tetrahydrofuran aufgenommen. 5-µl Proben werden auf Silicagel Si-60 Platten (Merck) aufgetragen. Die Chromatographie wird mit 78 ml Diethylether/22 ml Petroläther/1 ml Eisessig als Laufmittel durchgeführt. Die Menge an freigesetzter fluoreszierende NBD-Fettsäure wird durch Phosphorimaging (Molecular Dynamics, Storm 840 und ImageQuant Software) bei einer Anregungswellenlänge von 460 nm und Emissionswellenlänge von 540-560 nm bestimmt.

### Enzympräparation:

### Präparation der partiell gereinigten HSL:

Isolierte Rattenfettzellen werden aus Nebenhodenfettgewebe von nicht-behandelten männlichen Ratten (Wistar, 220-250 g) durch Kollagenasebehandlung gemäß publizierter Verfahren gewonnen (z.B. S. Nilsson et al., Anal. Biochem. 158, 1986, 399 - 407; G. Fredrikson et al., J. Biol. Chem. 256, 1981, 6311 - 6320; H. Tomquist et al., J. Biol. Chem. 251, 1976, 813 - 819). Die Fettzellen aus 10 Ratten werden dreimal durch Flotation mit jeweils 50 ml Homogenisationspuffer (25 ml Tris/HCl, pH 7.4, 0.25 M Sucrose, 1 mM EDTA, 1 mM DTT, 10 µg/ml Leupeptin, 10 µg/ml Antipain, 20 µg/ml Pepstatin) gewaschen und schließlich in 10 ml Homogenisationspuffer aufgenommen. Die Fettzellen werden im Teflon-in-Glas Homogenisator (Braun-Melsungen) durch 10 Hübe bei 1500 rpm und 15°C homogenisiert. Das Homogenisat wird zentrifugiert (Sorvall SM24-Röhrchen, 5000 rpm, 10 min, 4°C). Der Unterstand zwischen der oben liegenden Fettschicht und dem Pellet wird abgenommen und die Zentrifugation wiederholt. Der daraus resultierende Unterstand wird erneut zentrifugiert (Sorvall SM24-Röhrchen, 20000 rpm, 45 min, 4°C). Der Unterstand wird abgenommen und mit 1 g Heparin-Sepharose (Pharmacia-Biotech, CL-6B, 5 x gewaschen mit 25 mM Tris/HCl, pH 7.4, 150 mM NaCl) versetzt. Nach Inkubation für 60 min bei 4°C (in Intervallen von 15 min aufschütteln) wird der Ansatz zentrifugiert (Sorvall SM24-Röhrchen, 3000 rpm, 10 min, 4°C). Der Überstand wird durch Zugabe von Eisessig auf pH 5.2 gebracht und 30 min bei 4°C inkubiert. Die Präzipitate werden durch Zentrifugation (Sorvall SS34, 12000 rpm, 10 min, 4°C) gesammelt und in 2.5 ml 20 mM Tris/HCl, pH 7.0, 1 mM EDTA, 65 mM NaCl, 13 % sucrose, 1 mM DTT, 10 µg/ml Leupeptin/Pepstatin/Antipain suspendiert. Die Suspension wird über Nacht bei 4°C gegen 25 mM Tris/HCl, pH 7.4, 50 % Glyzerin, 1 mM DTT, 10 µg/ml Leupeptin, Pepstatin, Antipain dialysiert und dann auf eine Hydroxiapatit-Säule aufgetragen (0.1 g pro 1 ml Suspension, äquilibriert mit 10 mM Kaliumphosphat, pH 7.0, 30 % Glyzerin, 1 mM DTT). Die Säule wird mit vier Volumina Äquilibrierungspuffer bei einer Flußrate von 20 bis 30 ml/h gewaschen. Die HSL wird mit einem Volumen Äquilibrierungspuffer, der 0.5 M Kaliumphosphat enthält, eluiert, sodann dialysiert (s.o.) und 5- bis 10-fach konzentriert durch Ultrafiltration (Amicon Diaflo PM 10 Filter) bei 4°C. Die partiell gereinigte HSL kann 4 bis 6 Wochen bei -70°C aufbewahrt werden.

### Assay:

Für die Herstellung des Substrats werden 25-50 µCi [3H]Trioleoylglycerin (in Toluol), 6.8 µMol unmarkiertes Trioleoylglycerin und 0.6 mg Phospholipide (Phosphatidylcholin/Phosphatidylinositol 3:1 w/v) gemischt, über N2 getrocknet und dann in 2 ml 0.1 M KPi (pH 7.0) durch Ultraschallbehandlung (Branson 250, Mikrospitze, Einstellung 1-2, 2 x 1 min im 1-min Intervall) aufgenommen. Nach Zugabe von 1 ml KPi und erneuter Ultraschallbehandlung (4 x 30 sec auf Eis in 30-sec Intervallen) wird 1 ml 20% BSA (in KPi) hinzugefügt (Endkonzentration Trioleoylglyzerin 1.7 mM). Für die Reaktion werden 100 µl Substratlösung zu 100 µl HSL-Lösung (HSL präpariert wie oben, verdünnt in 20 mM KPi, pH 7.0, 1 mM EDTA, 1 mM DTT, 0.02% BSA, 20 µg/ml Pepstatin, 10 µg/ml Leupeptin) pipettiert und für 30 min bei 37°C irikubiert. Nach Zugabe von 3.25 ml Methanol/Chloroform/Heptan. (10:9:7) und von 1.05 ml 0.1 M K2CO3, 0.1 M Borsäure (pH 10.5) wird gut gemischt und schließlich zentrifugiert (800 x g, 20 min). Nach Phasentrennung wird ein Äquivalent der oberen Phase (1 ml) abgenommen und die Radioaktivität durch Flüssigszintillationsmessung bestimmt.

### Auswertung:

Substanzen werden üblicherweise in vier unabhängigen Ansätzen geprüft. Die Hemmung der enzymatischen Aktivität der HSL durch eine Testsubstanz wird durch den Vergleich mit einer nicht-gehemmten Kontrollreaktion bestimmt. Die Berechnung des IC50-Wertes erfolgt über eine Hemmkurve mit mind. 10 Konzentrationen der Testsubstanz. Für die Analyse der Daten wird das Softwarepaket GRAPHIT, Elsevier-BIOSOFT, benutzt.

In diesem Test zeigten die Verbindungen der Beispiele 1 bis 55 Inhibitionen im Bereich von IC₅₀ 0,04-5 µM.

Die nachfolgenden Beispiel beschreiben die Erfindung näher, ohne sie einzuschränken.

### Beispiele:

Die folgenden Beispiel wurden nach den im folgenden beschriebenen Methoden hergestellt:

### Methode A:

Zu einer Lösung von 2mmol 1H-Benzotriazol in Pyridin (5mL) und Dichlormethan (10 mL) wird eine Lösung des entsprechenden Carbamoylchlorids (1 mmol) in Dichlormethan (10 mL) zugegeben. Das Reaktionsgemisch wird 16 h bei RT gerührt, dann mit EtOAc (15 mL) versetzt, über Kieselgel filtriert und das Filtrat eingeengt. Das Produkt wird durch präparative HPLC gereinigt und gefriergetrocknet.

Methode B:

### a) Herstellung einer Benzotriazol-1-carbonsäurechlorid-Lösung

Zu einer Phosgenlösung (20% in Toluol; 90 mL; 182 mmol) tropft man unter Eiskühlung eine Lösung von Benzotriazol (6 g, 50,4 mmol) in THF (100 mL) zu. Das Eisbad wird entfernt und die Lösung noch 2 h bei RT gerührt. Das Lösungmittel wird abdestilliert und der Rückstand in THF zu einem Gesamtvolumen von 25 mL aufgenommen.

### b) Umsetzung der Benzotriazolcarbonsäurechloride zu den entsprechenden Benzotriazol-1-carbonsäureamiden und aniliden

Jeweils 10 Amine bzw. Aniline (2 mmol) werden in THF (1 mL) vorgelegt und mit Pyridin ( 0,2 mL) versetzt. Die Ansätze werden mit Benzotriazol-1-carbonsäurechlorid-Lösung (1 mL, - 2 mmol) inkubiert und 16 h bei RT gerührt. Anschließend werden die Ansätze mit Ethylacetat verdünnt (5mL), über Kieselgel filtriert und das Filtrat im Vakuum zur Trockne eingeengt. Die Rohprodukte werden durch Flash Chromatographie gereinigt.

### Beispiel 1:

### 3-(4-Methyl-piperidin-1-carbonyl)-3H-benzotriazol-5-carbonsäuremethylester

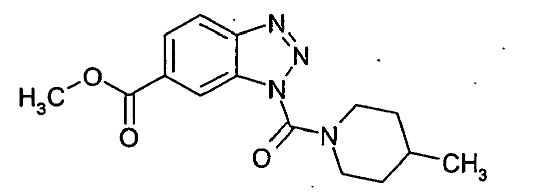

M+H+: 303,14

### Beispiel 2:

### (8-Aza-spiro[4.5]dec-8-yl)-benzotriazol-1-yl-methanon

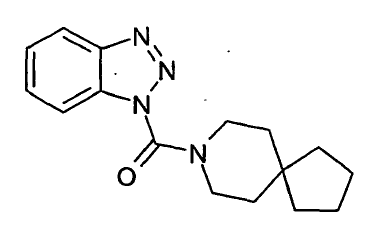

M+H+: 285,16

### Beispiel 3:

### Benzotriazol-1-yl-(6,7-dimethoxy-3,4-dihydro-1H-isochinolin-2-yl)-methanon

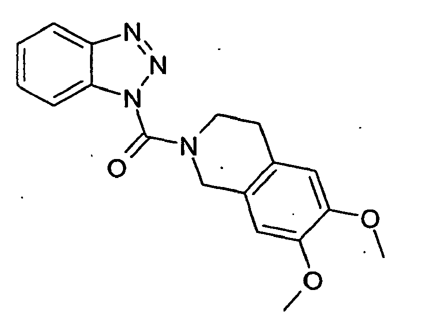

M+H+: 339,13

### Beispiel 4:

### (5-Phenoxy-benzotriazol-1-yl)-(4-trifluoromethyl-piperidin-1-yl)-methanon

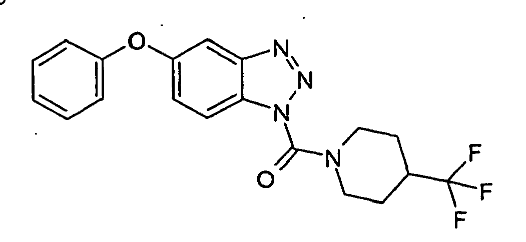

M+H+: 391,13

### Beispiel 5:

### (6-Chlor-5-fluor-benzotriazol-1-yl)-(4-methyl-piperidin-1-yl)-methanon

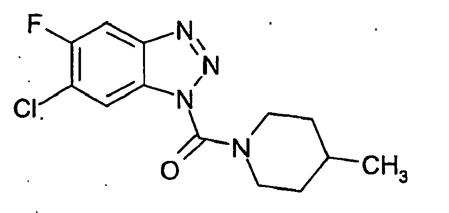

M+H+:297,74

### Beispiel 6:

### Benzotriazol-1-yl-(4-brom-piperidin-1-yl)-methanon

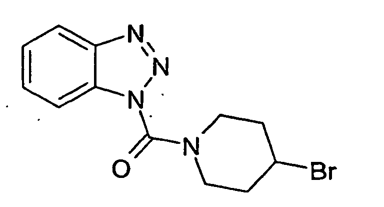

M+H+: 310,3

### Beispiel 7:

### Benzotriazol-1-yl-(4-trifiuoromethyl-piperidin-1-yl)-methanon

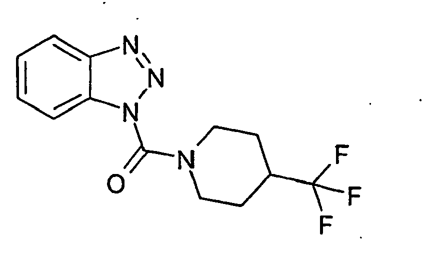

M+H+: 299,18

### Beispiel 8:

### Benzotriazol-1-yl-(1,3-dihydro-isoindol-2-yl)-methanon.

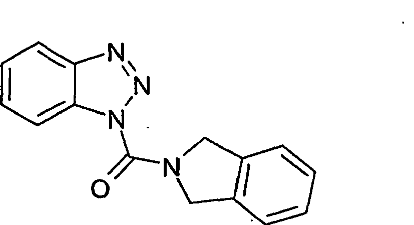

M+H+: 265.0

### Beispiel 9:

### 1-(3,6-Dihydro-2H-pyridin-1-carbonyl)-1H-benzotriazol-5-carbonsäure methylester

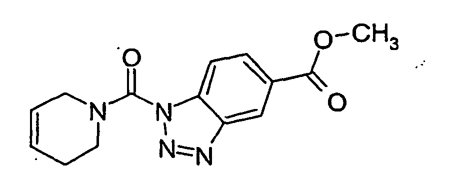

M+H+: 287,04

### Beispiel 10:

### (3,6-Dihydro-2H-pyridin-1-yl)-(5-nitro-benzotriazol-1-yl)-methanon

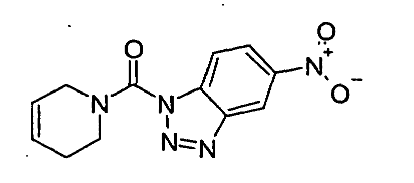

M+Na: 296,21

### Beispiel 11:

### (3,4-Dihydro-1 H-isochinolin-2-yl)-(5-nitro-benzotriazol-1-yl)-methanon

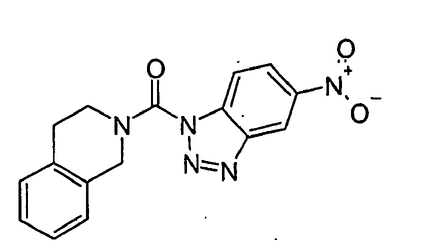

M+H+: 324,10

### Beispiel 12:

### (5-Brom-benzotriazol-1-yl)-(3,6-dihydro-2H-pyridin-1-yl)-methanon

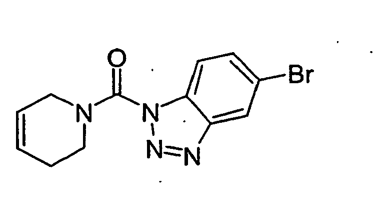

M+H+: 306,98

### Beispiel 13:

### (5-Brom-benzotriazol-1-yl)-(3-trifluoromethyl-piperidin-1-yl)-methanon

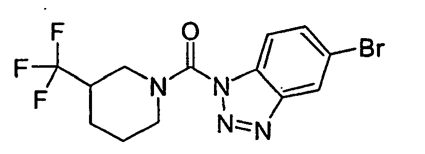

M+H+: 377,30

### Beispiel 14:

### (5-Brom-benzotriazol-1-yl)-(3,4-dihydro-1H-isochinolin-2-yl)-methanon

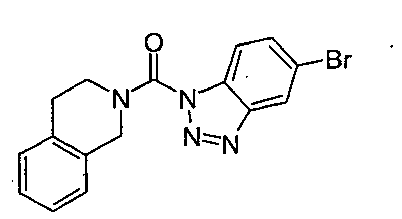

M+H+: 357,04

### Beispiel 15:

### Benzotriazol-1-yl-(octahydro-isoindol-2-yl)-methanon

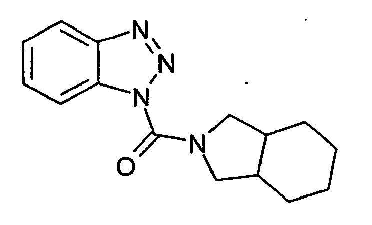

M+H+: 271.15

### Beispiel 16:

### (7-Amino-3,4-dihydro-1 H-isochinolin-2-yl)-benzotriazol-1-yl-methanon

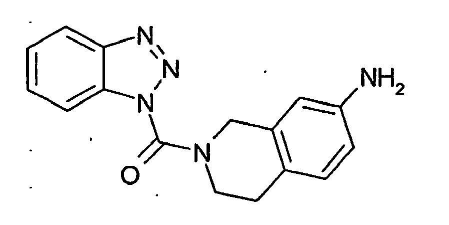

M+H+: 294.0

### Beispiel 17:

### (3,4-Dihydro-1 H-isochinolin-2-yl)-(5-methoxy-benzotriazol-1-yl)-methanon

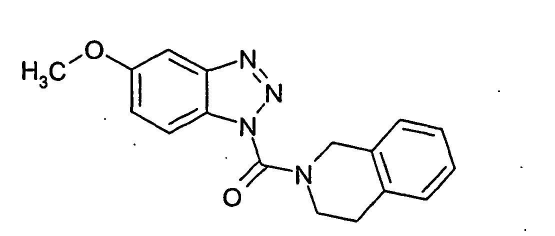

M+H+:309,04

### Beispiel 18:

### (5-Methoxy-benzotriazol-1-yl)-(3-methyl-piperidin-1-yl)-methanon

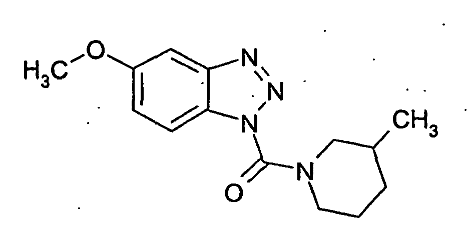

M+H+: 275,5

### Beispiel 19:

### (6-Amino-benzotriazol-1-yl)-(4-methyl-piperidin-1-yl)-methanon

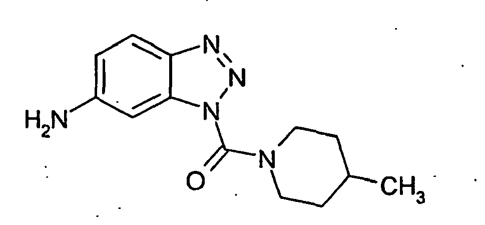

M+H+: 260,1

### Beispiel 20:

### (5-Chlor-benzotriazol-1-yl)-(4-methyl-piperidin-1-yl)-methanon

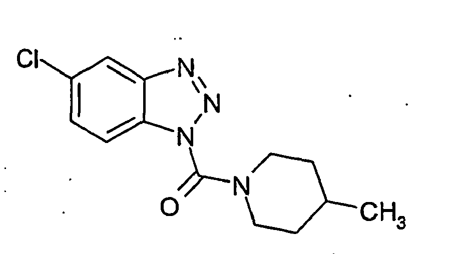

M+H+: 279,6

### Beispiel 21:

### Benzotriazol-1-yl-(1,2,6-triaza-spiro[2.5]oct-1-en-6-yl)-methanon

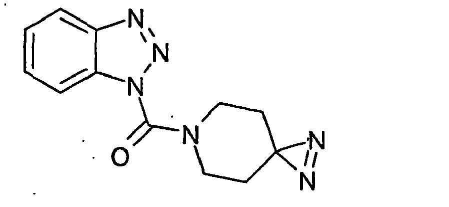

M+Na+: 279,19

### Beispiel 22:

### Benzotriazol-1-yl-(4-ethyl-piperidin-1-yl)-methanon

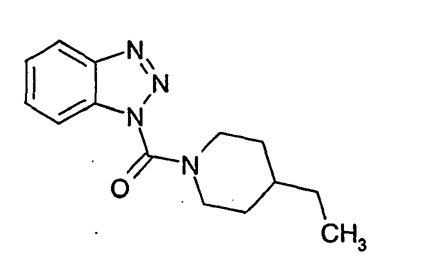

M+H+: 259,04

### Beispiel 23:

### (4-Methyl-piperidin-1-yl)-(6-nitro-benzotriazol-1-yl)-methanon

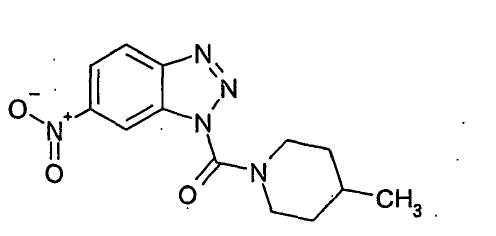

M+H+: 290,4

### Beispiel 24:

### 1-(Benzotriazol-1-carbonyl)-piperidine-4-carbonsäureethylester

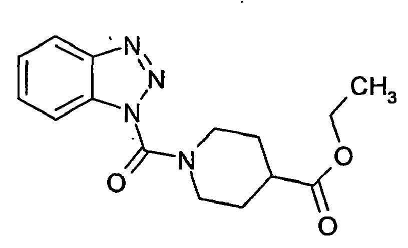

M+H+:303,13

### Beispiel 25:

### Benzotriazol-1-yl-(4-methyl-piperidin-1-yl)-methanon

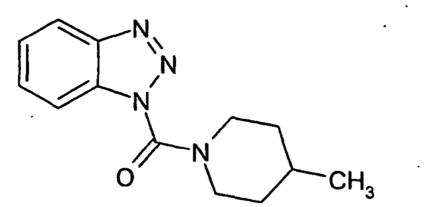

M+H+: 245,0

### Beispiel 26:

### 3-(4-Methyl-piperidin-1-carbonyl)-3H-benzotriazol-5-carbonitril

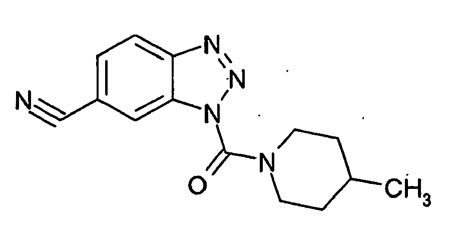

M+H+: 270,12

### Beispiel 27:

### Benzotriazol-1-yl-(3,4-dihydro-1H-isochinolin-1-yl)-methanon

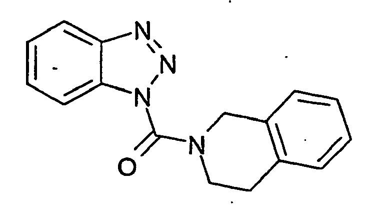

M+H+: 279,11

### Beispiel 28:

### Benzotriazol-1-yl-(3,4-dihydro-2H-chinolin-1-yl)-methanon

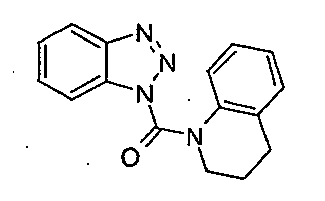

M+H+: 279,2

### Beispiel 29:

### (6-Methyl-benzotriazol-1-yl)-pyrrolidin-1-yl-methanon

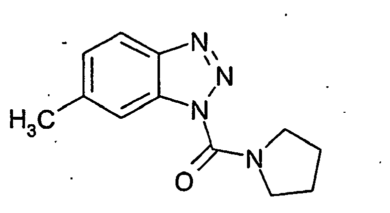

M+H+: 231,11

### Beispiel 30:

### Benzotriazol-1-yl-(3-methyl-piperidin-1-yl)-methanon

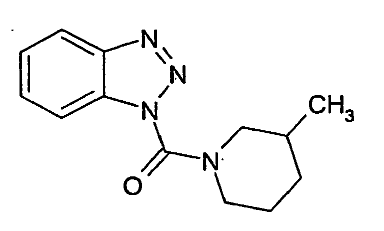

M+H+: 245,13

### Beispiel 31:

### Benzotriazol-1-yl-(3,4-dimethyl-piperidin-1-yl)-methanon

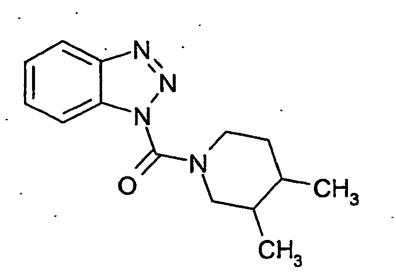

M+H+: 259,14

### Beispiel 32:

### [1-(4-Methyl-piperidin-1-carbonyl)-1H-benzotriazol-5-yl]-phenyl-methanon

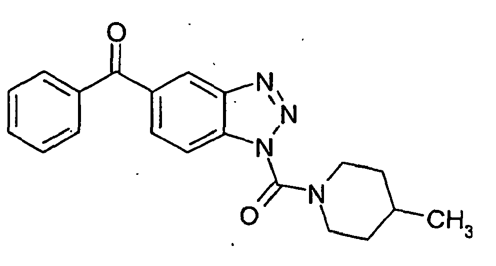

M+H+: 349,15

### Beispiel 33:

### (4-Methyl-piperidin-1-yl)-(5-trifluormethyl-benzotriazol-1-yl)-methanon

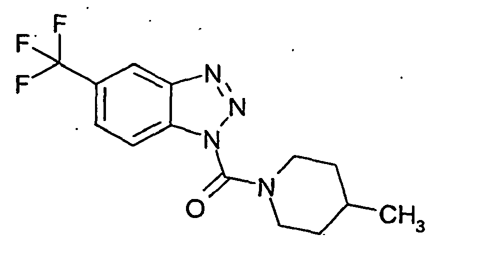

M+H+: 313,5

### Beispiel 34:

### (6-Brom-benzotriazol-1-yl)-(4-methyl-piperidin-1-yl)-methanon

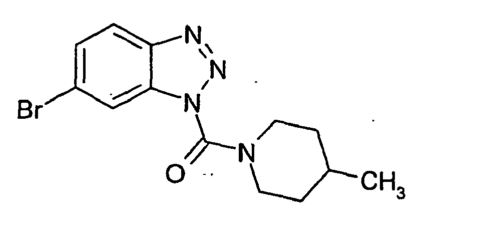

M+H+: 324,0

### Beispiel 35:

### Benzotriazol-1-yl-(4-tert-butyl-piperidin-1-yl)-methanon

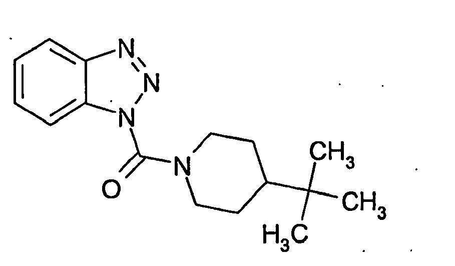

M+H+: 287,17

### Beispiel 36:

### [5-(Hydroxy-phenyl-methyl)-benzotriazol-1-yl]-(4-methyl-piperidin-1-yl)- methanon

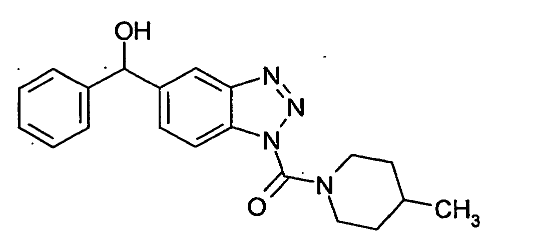

M+H+: 350,17

### Beispiel 37:

### (4-Methyl-piperidin-1-yl)-(5-phenoxy-benzotriazol-1-yl)-methanon

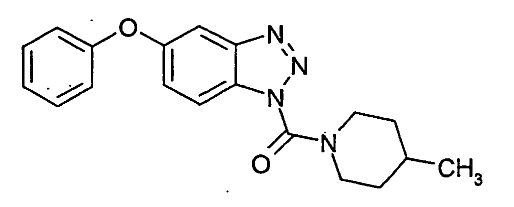

M+H+: 337,3

### Beispiel 38:

### (5-Cyclohexylsulfanyl-benzotriazol-1-yl)-(4-methyl-piperidin-1-yl)-methanon

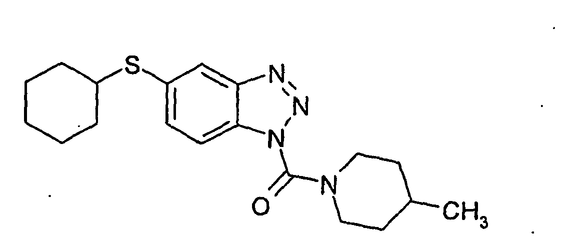

M+H+: 359,17

### Beispiel 39:

### Benzotriazol-1-yl-(4-isopropyl-piperidin-1-yl)-methanon

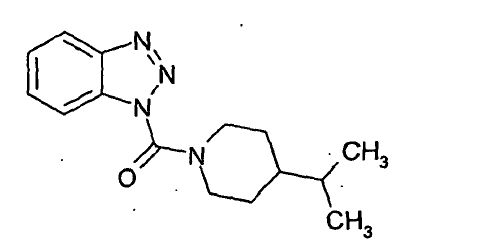

M+H+: 273,3

### Beispiel 40:

### (6-Chlor-benzotriazol-1-yl)(4-methyl-piperidin-1-yl)methanon

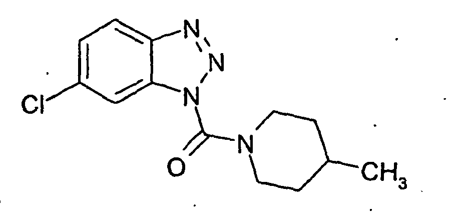

M+H+: 279,5

### Beispiel 41:

### Benzotriazol-1-yl-(6-methoxy-3,4-dihydro-1H-isochinolin-2-yl)-methanon

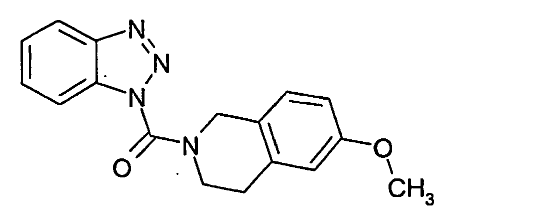

M+H+: 309,3

### Beispiel 42:

### Benzotriazol-1-yl-(4-benzyl-piperidin-1-yl)-methanon

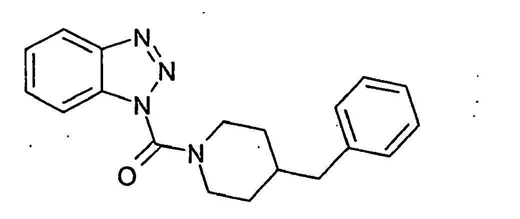

M+H+: 321.1

### Beispiel 43:

### (5-Brom-benzotriazol-1-yl)-(3-methyl-piperidin-1-yl)-methanon

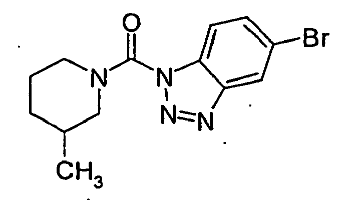

M+H+: 325,31

### Beispiel 44:

### 1-(3,4-Dihydro-1H-isochinoline-2-carbonyl)-1H-benzotriazol-5-carbonitril

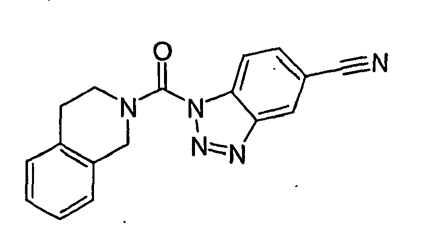

M+H+: 270,12

### Beispiel 45:

### (4-Chlor-6-trifluoromethyl-benzotriazol-1-yl)-(3,4-dihydro-1H-isochinotin-2-yl) methanon

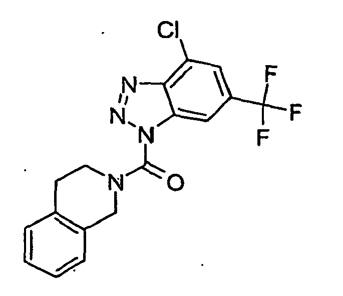

M+H+: 381.06

### Beispiel 46:

### 1-(3,4-Dihydro-2H-chinolin-1-carbonyl)-1H-benzotriazol-5-carbonitril

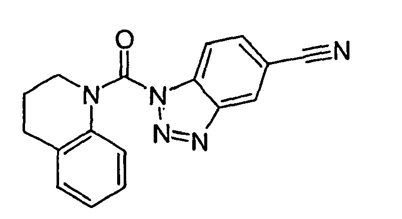

M+H+: 304,11

### Beispiel 47:

### (4-Chlor-6-trifluoromethyl-benzotriazol-1-yl)-(3,6-dihydro-2H-pyridin-1-yl)- methanon

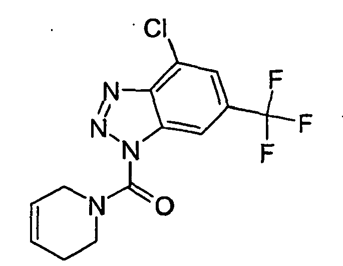

M+H+: 331,04

### Beispiel 48:

### Benzotriazol-1-yl-(7-methoxy-3,4-dihydro-1H-isochinolin-2-yl)-methanon

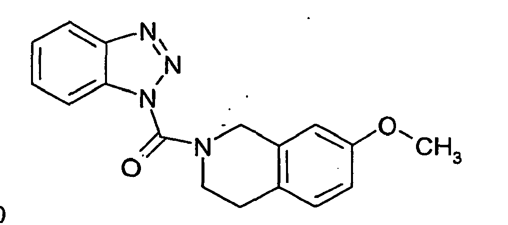

M+H+: 309.1

### Beispiel 49:

### (5-Methoxy-benzotriazol-1-yl)-(4-methyl-piperidin-1-yl)-methanon

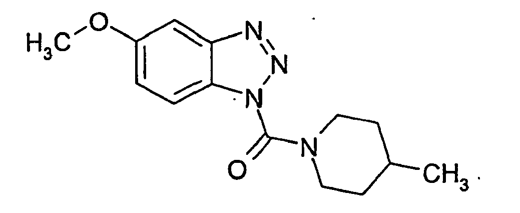

M+H+: 275,3

### Beispiel 50:

### (3,4-Dihydro-1H-isochinolin-2-yl)-(6-nitro-benzotriazol-1-yl)-methanon

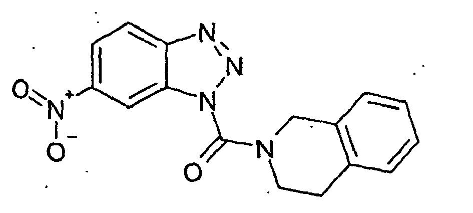

M+H+: 324,3

### Beispiel 51:

### (6-Benzoyl-benzotriazol-1-yl)-(4-methyl-pipendin-1-yl)-methanon

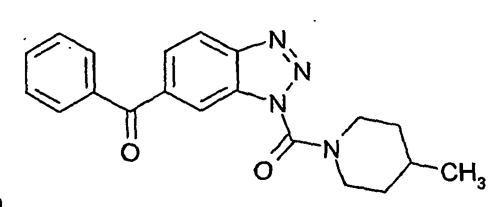

M+H+: 349,15

### Beispiel 52:

### Benzotriazol-1-yl-(7-nitro-3,4-dihydro-1H-isochinolin-2-yl)-methanon

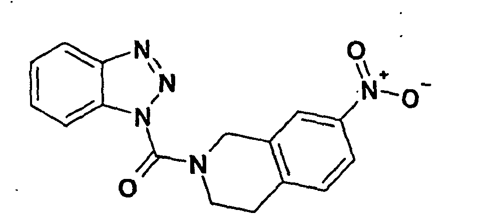

M+H+: 324.1

### Beispiel 53:

### Azepan-1-yl-benzotriazol-1-yl-methanon

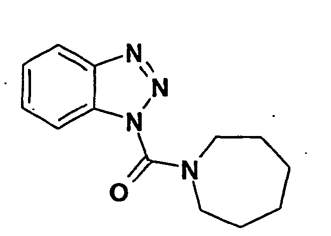

M+H+: 245.3

### Beispiel 54:

### Benzotriazol-1-yl-(4-chlor-piperidin-1-yl)-methanon

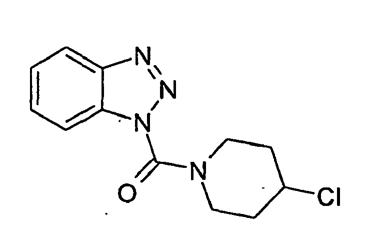

M+H+:265,7

### Beispiel 55:

### Benzotriazol-1-yl-(3,6-dihydro-2H-pyridin-1-yl)-methanon

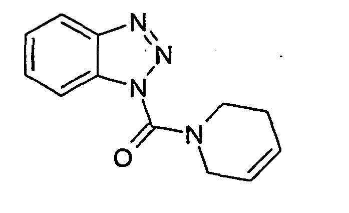

M+H+: 229,2.

## Patentansprüche

1. Benzotriazole der allgemeinen Formel I, 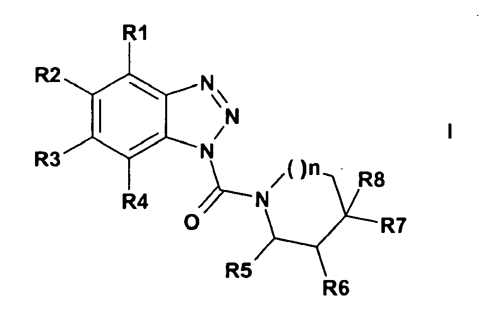 worin bedeuten :
R1 bis R8 H,
wobei einer dieser Reste R2 oder R3 stehen kann für:
Br, Cl, CH₃, CN, NH₂, NO₂, CF₃, OCH₃, Phenoxy, Benzoyl, CH(OH)-Phenyl, S-Cyclohexyl, CO-OCH₃;
oder
zwei Substituenten aus dieser Reihe sind:
R1 = Cl und R3 = CF₃ oder
R2 = F und R3 = Cl;
n eine ganze Zahl von 0, 1 oder 2; und
einer der Substituenten R6 oder R7 stehen kann für :
R6 CH₃;
R7 CH₃, C₂H₅; CH(CH₃)₂, C(CH₃)₃, CF₃, Br, Cl, Benzyl oder CO- OC₂H₅; oder
R6 und R7 beide CH₃ sind; oder
der Ring anstelle R6 und R7 eine Doppelbindung enthalten kann oder
R5 und R6 oder R6 und R7 zusammen mit den sie tragenden C-Atomen für einen benzannellierten Ring oder falls n = 0 ist, auch für Cyclohexandiyl stehen kann; wobei im Falle des Ringschlussses R6/R7 dieser Substituent gegebenenfalls einfach durch NH₂ oder NO₂ oder ein- bis zweifach durch OCH₃ substituiert sein kann; und
R7 und R8 zusammen Cyclopentyl, Diazirin oder =CH₂;
wobei die Verbindungen mit R1 bis R5 und R8 =H, n = 1 und R6/R7 = benzannelliert und R1, R3-R8 = H, R2 = CH₃ und n = 1 ausgenommen sind.

2. Benzotriazole der allgemeinen Formel I gemäß Anspruch 1,
worin bedeuten :
R1 bis R8 H;
wobei einer dieser Reste R2 oder R3 stehen kann für:
R2 Br, Cl, CN, NO₂, CF₃, OCH₃, Phenoxy, Benzoyl, CH(OH)-Phenyl, S-Cyclohexyl, CO-OCH₃;
R3 CH₃, CN, Br, Cl, NH₂, NO₂, Benzoyl

3. Benzotriazole der allgemeinen Formel I gemäß Anspruch 1 oder 2, worin bedeuten:
R1 bis R8 H;
wobei einer dieser Reste R2 oder R3 stehen kann für:
R2 Br, Cl, NO₂, OCH₃, Phenoxy, CO-OCH₃;
R3 NH₂; oder
zwei Substituenten aus dieser Reihe sind:
R2 = F und R3 = Cl;
n eine ganze Zahl von 1 oder 2; und
einer der Substituenten R6 oder R7 stehen kann für :
R6 CH₃;
R7 CH₃, CF₃ oder Br; oder
der Ring anstelle R6 und R7 eine Doppelbindung enthalten kann oder
R6 und R7 zusammen mit den sie tragenden C-Atomen für einen benzannellierten Ring stehen kann, der gegebenenfalls einfach durch NH₂ oder einbis zweifach durch OCH₃ substituiert sein kann; und
R7 und R8 zusammen Cyclopentyl oder
n eine ganze Zahl von 0; und
R6 und R7 zusammen mit den sie tragenden C-Atomen für einen benzannellierten Ring oder Cyclohexandiyl stehen kann.

4. Benzotriazole der allgemeinen Formel I gemäß den Ansprüchen 1 bis 2, worin
R1 bis R8 H;
wobei einer dieser Reste R2 oder R3 stehen kann für:
R2 Br, CN, CF₃, OCH₃, Phenoxy, Benzoyl, CH(OH)-Phenyl, S-Cyclohexyl;
R3 CN, Br, Cl, NO₂, Benzoyl; oder
zwei Substituenten aus dieser Reihe sind:
R1 = Cl und R3 = CF₃;
n eine ganze Zahl von 1; und
einer der Substituenten R6 oder R7 stehen kann für :
R6 CH₃;
R7 CH₃, C₂H₅; CH(CH₃)₂, C(CH₃)₃, Benzyl oder CO-OC₂H₅; oder
R6 und R7 beide CH₃ sind; oder
der Ring anstelle R6 und R7 eine Doppelbindung enthalten kann oder
R5 und R6 oder R6 und R7 zusammen mit den sie tragenden C-Atomen für einen benzannellierten Ring stehen kann;
wobei die Verbindungen mit R1 bis R5 und R8 =H, n = 1 und R6/R7 = benzannelliert ausgenommen ist.

5. Benzotriazole gemäß Anspruch 1 der folgenden Strukturen:

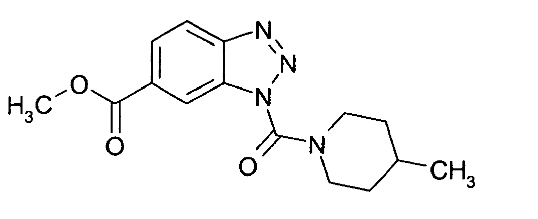

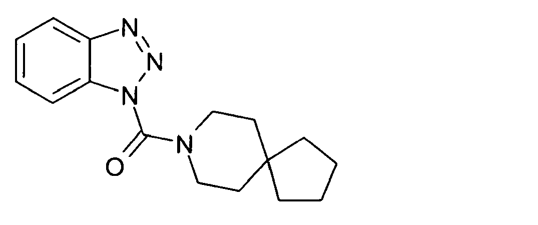

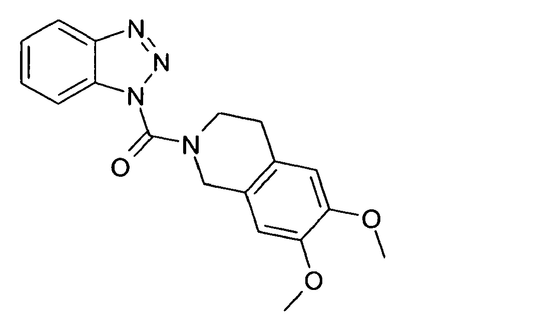

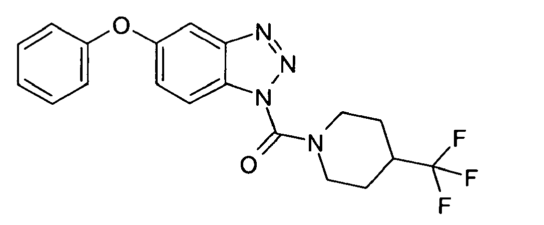

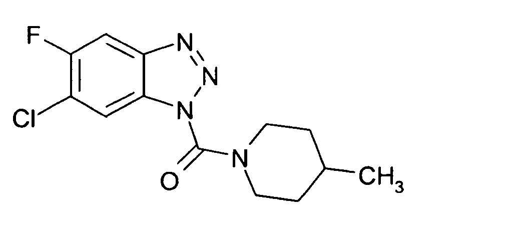

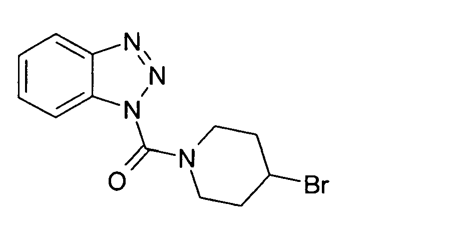

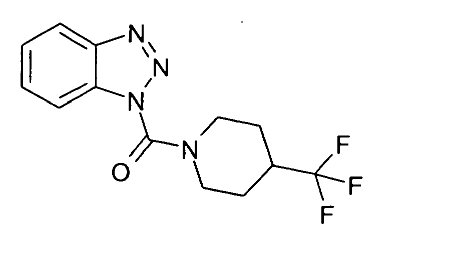

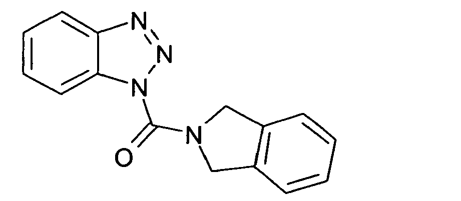

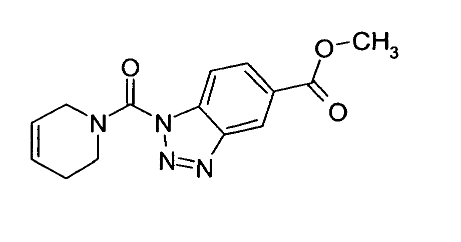

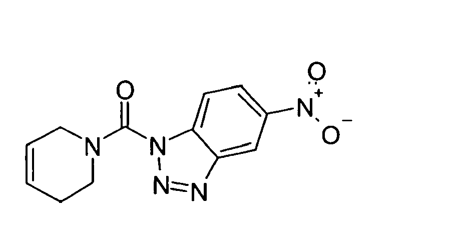

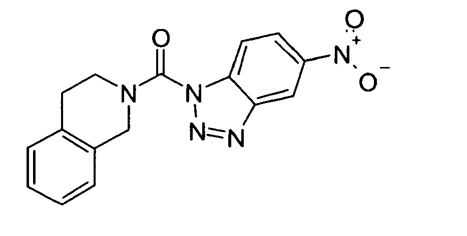

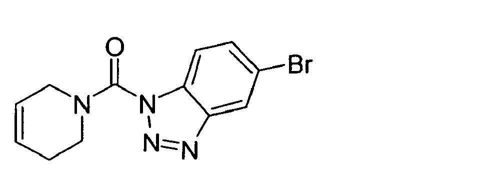

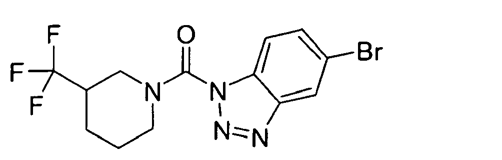

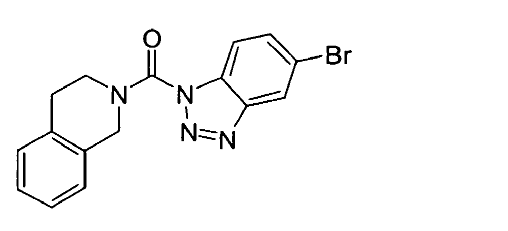

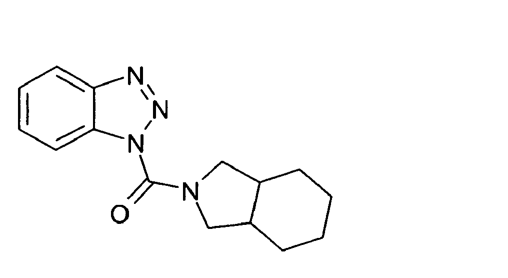

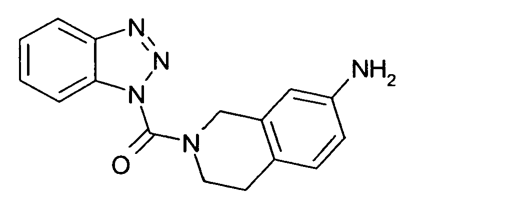

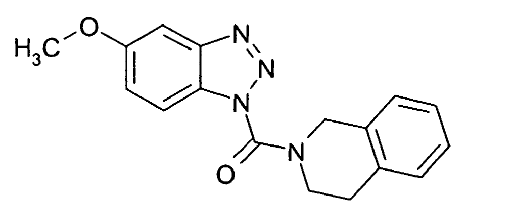

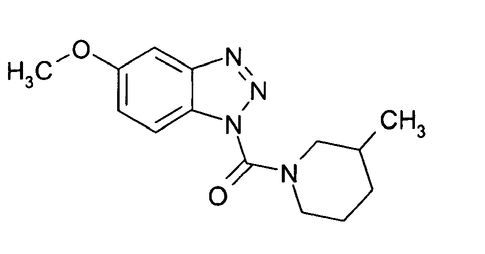

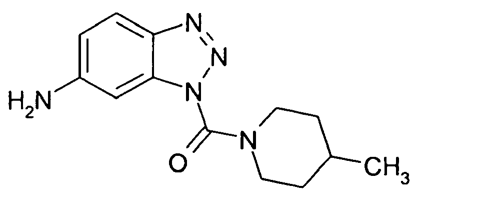

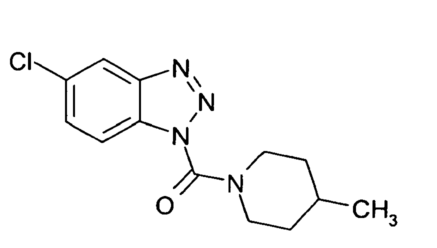

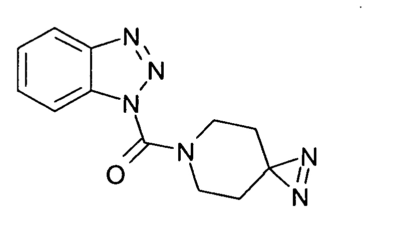

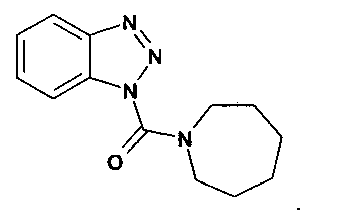

6. Benzotrialzole gemäß Anspruch 1 der folgenden Strukturen

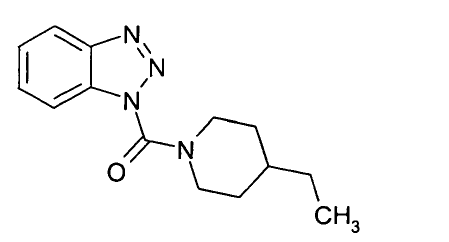

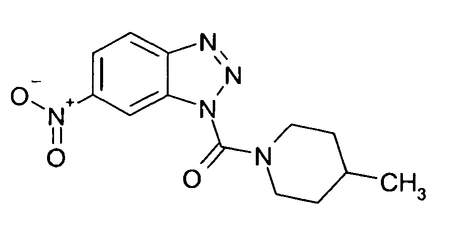

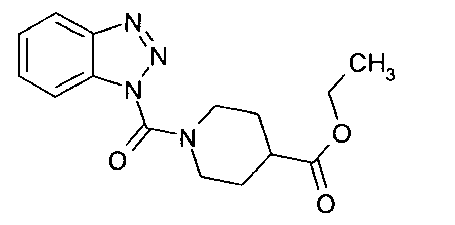

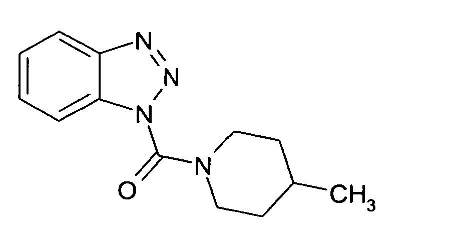

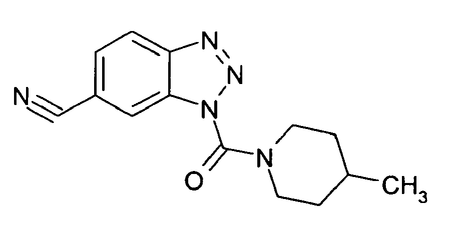

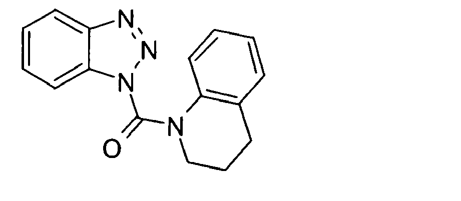

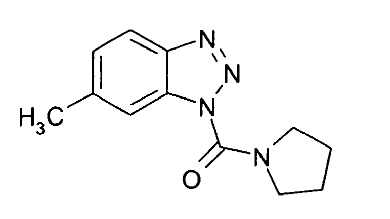

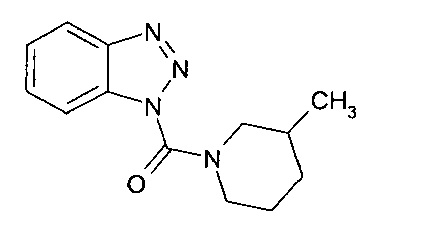

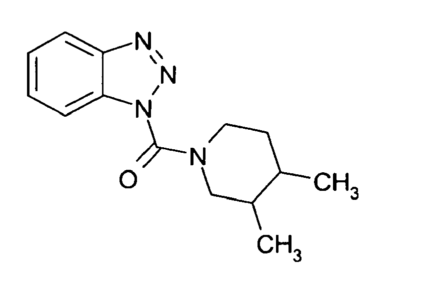

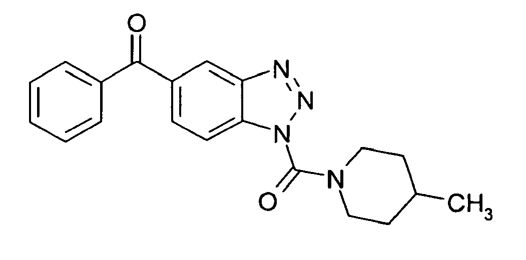

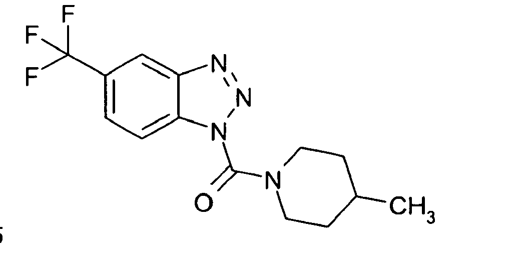

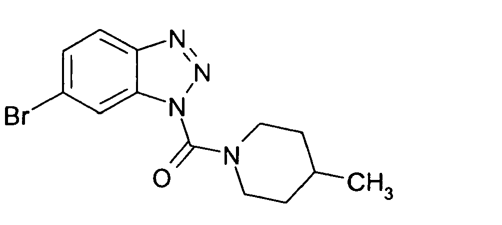

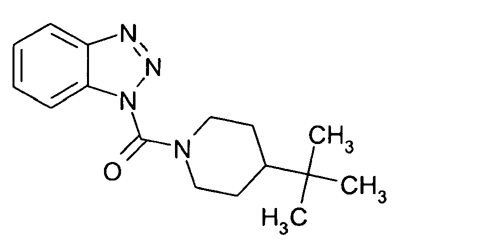

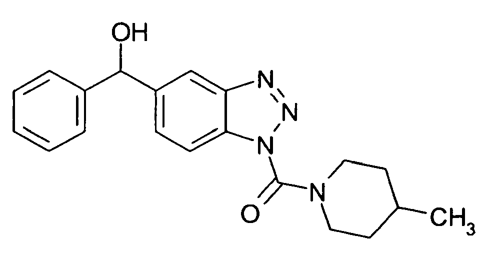

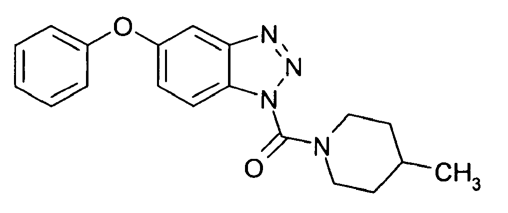

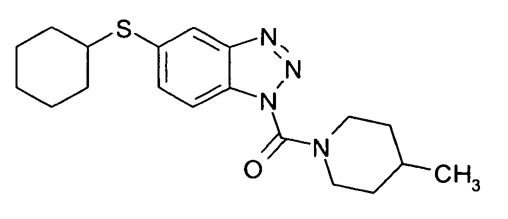

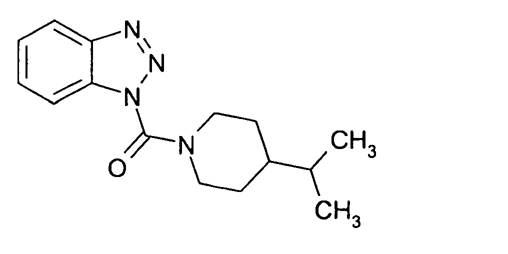

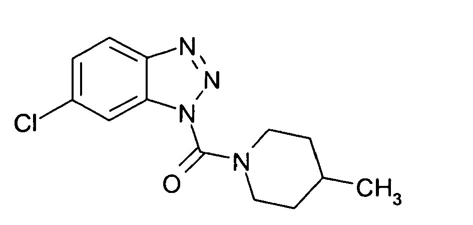

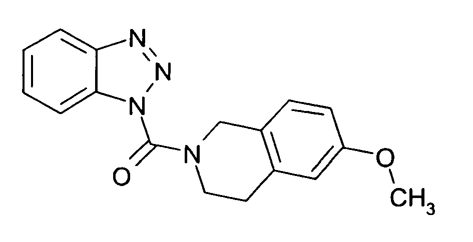

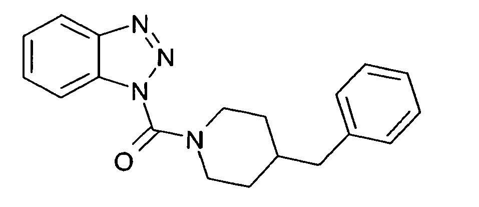

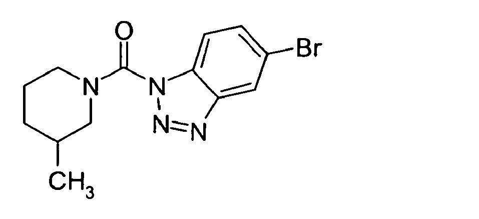

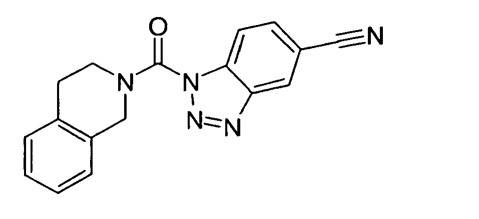

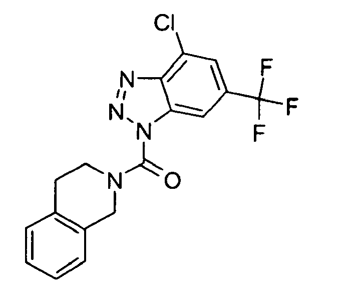

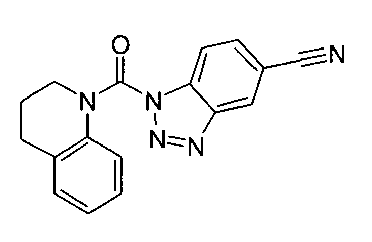

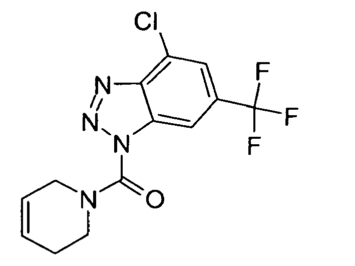

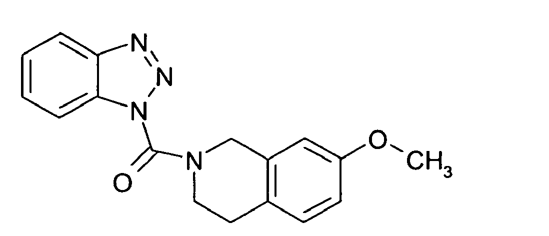

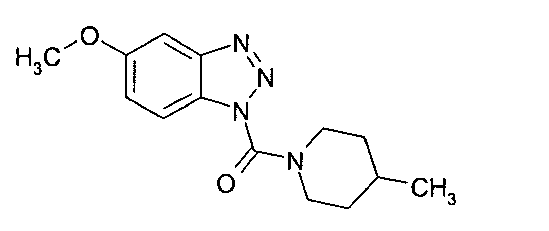

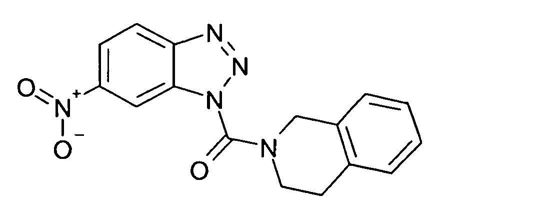

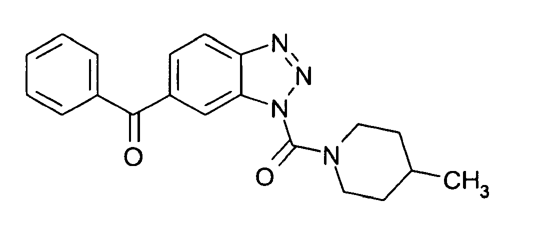

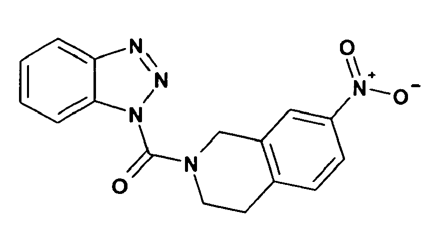

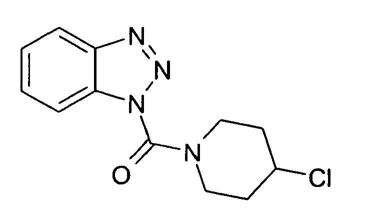

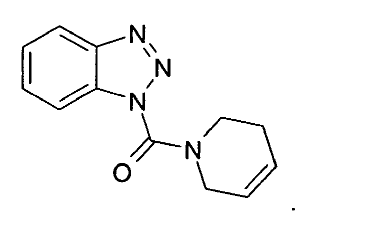

7. Benzotriazole der folgenden Strukturen gemäß Anspruch 6:

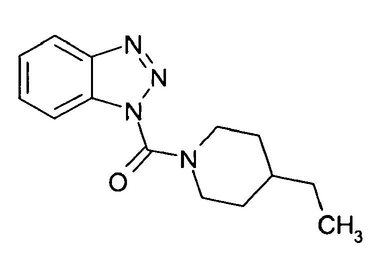

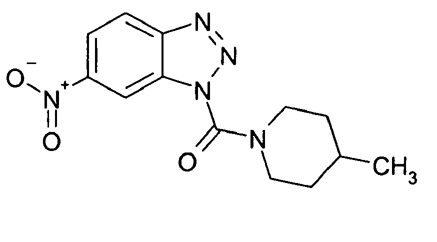

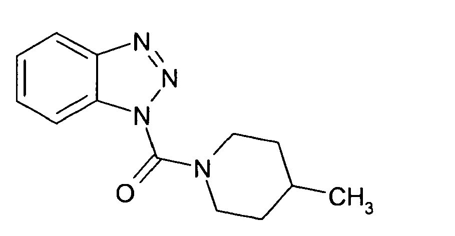

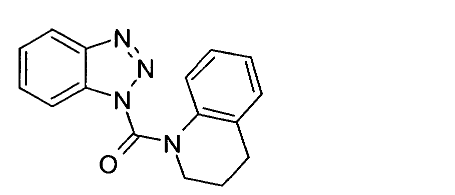

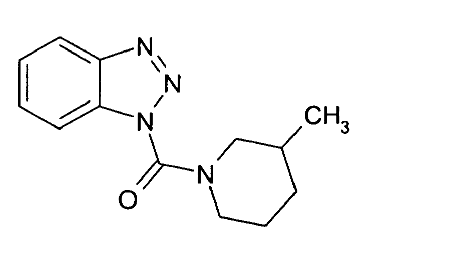

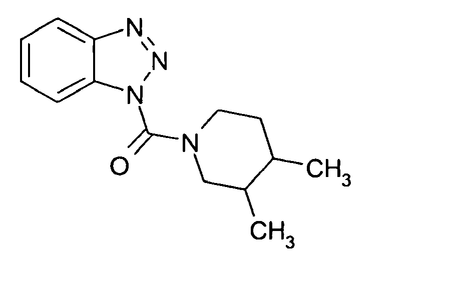

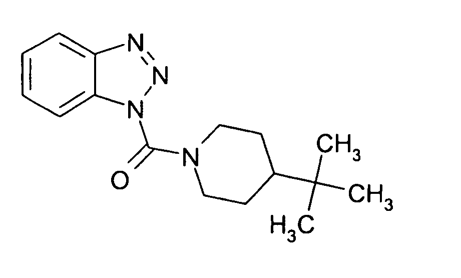

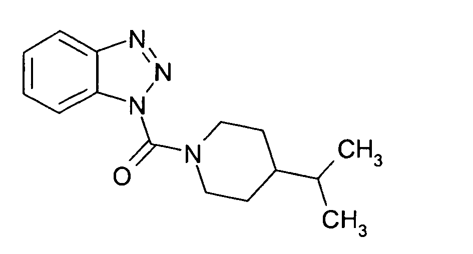

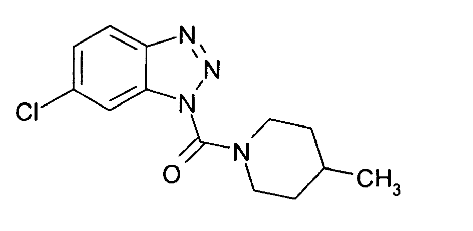

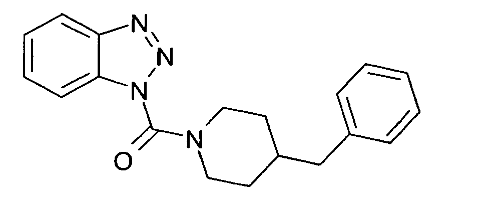

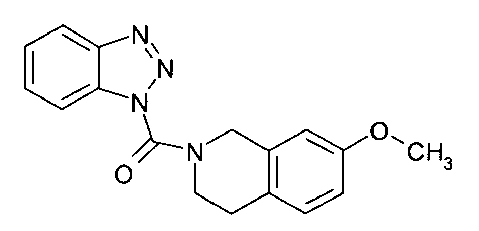

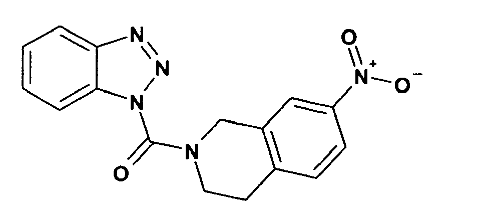

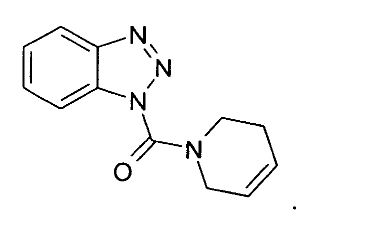

8. Benzotriazole der folgenden Strukturen gemäß Anspruch 7:

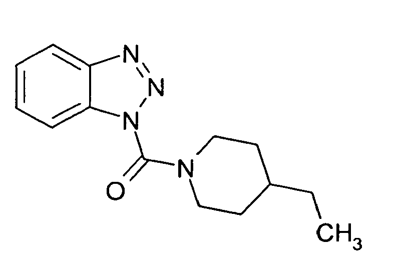

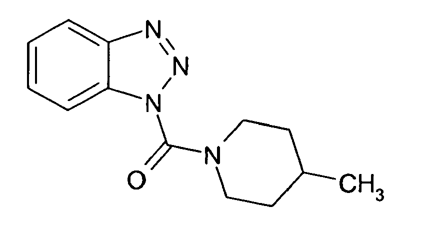

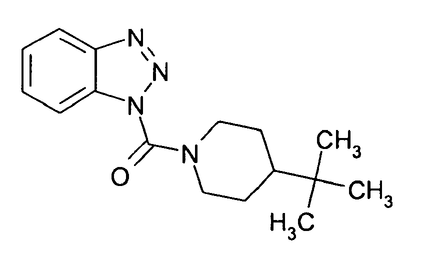

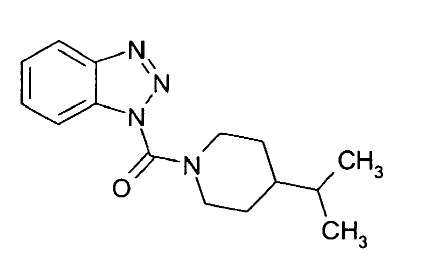

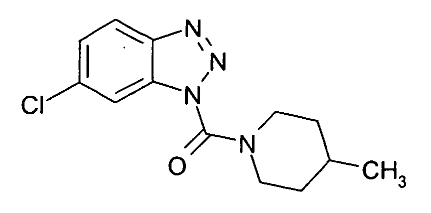

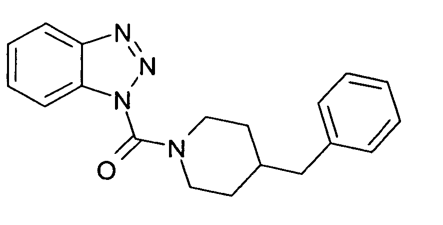

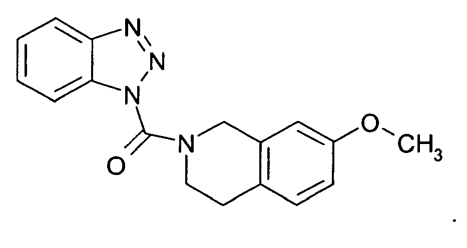

9. Benzotriazole der allgemeinen Formel I, 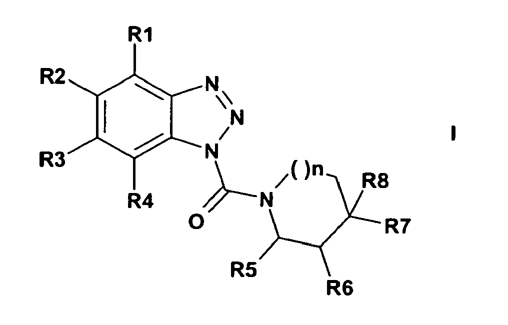 worin bedeuten :
R1 bis R8 H;
wobei einer dieser Reste R2 oder R3 stehen kann für:
Br, Cl, CH₃, CN, NH₂, NO₂, CF₃, OCH₃, Phenoxy, Benzoyl, CH(OH)-Phenyl, S-Cyclohexyl, CO-OCH₃;
oder
zwei Substituenten aus dieser Reihe sind:
R1 = Cl und R3 = CF₃ oder
R2 = F und R3 = Cl;
n eine ganze Zahl von 0, 1 oder 2; und
einer der Substituenten R6 oder R7 stehen kann für :
R6 CH₃;
R7 CH₃, C₂H₅; CH(CH₃)₂, C(CH₃)₃, CF₃, Br, Cl, Benzyl oder CO- OC₂H₅; oder
R6 und R7 beide CH₃ sind; oder
der Ring anstelle R6 und R7 eine Doppelbindung enthalten kann oder
R5 und R6 oder R6 und R7 zusammen mit den sie tragenden C-Atomen für einen benzannellierten Ring oder falls n = 0 ist, auch für Cyclohexandiyl stehen kann; wobei im Falle des Ringschlusses R6/R7 dieser substituent gegebenenfalls einfach durch NH₂ oder NO₂ oder ein- bis zweifach durch OCH₃ substituiert sein kann; und
R7 und R8 zusammen Cyclopentyl, Diazirin oder =CH₂;
zur Anwendung in einem Arzneimittel.

10. Benzotriazole der Formel 1 gemäß Anspruch 9 zur Anwendung in einem Arzneimittel mit hemmender Wirkung an der hormonsensitiven Lipase, HSL.

11. Benzotriazole der Formel I gemäß Anspruch 9 zur Anwendung in einem Arzneimittel zur Behandlung des nicht insulinabhängigen Diabetes Mellitus, des diabetischen Syndroms oder Syndrom X.

12. Arzneimittel zur Behandlung des nicht insulinabhängigen Diabetes Mellitus oder des diabetischen Syndroms enthaltend mindestens ein Benzotriazol der Formel I gemäß Anspruch 9.

## Claims

1. Benzotriazoles of the general formula I, 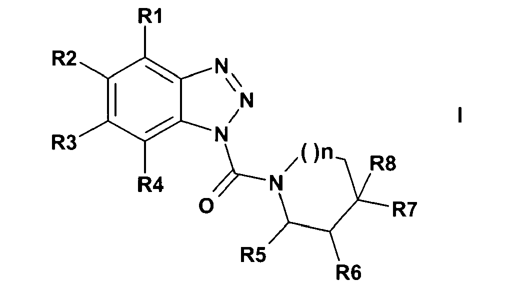 in which the meanings are:
R1 to R8 H,
where one of these radicals R2 or R3 may represent:
Br, Cl, CH₃, CN, NH₂, NO₂, CF₃, OCH₃, phenoxy, benzoyl, CH(OH)-phenyl, S-cyclohexyl, CO-OCH₃ ;
or
two substituents of this series are:
R1 = Cl and R3 = CF₃ or
R2 = F and R3 = Cl;
n an integer from 0, 1 or 2; and
one of the substituents R6 or R7 may represent:
R6 CH₃ ;
R7 CH₃, C₂H₅; CH(CH₃)₂, C(CH₃)₃, CF₃, Br, Cl, benzyl or CO-OC₂H₅; or
R6 and R7 are both CH₃; or
the ring may contain a double bond instead of R6 and R7 or
R5 and R6 or R6 and R7 may together with the carbon atoms carrying them represent a benzo-fused ring or else if n = 0 may represent cyclohexanediyl; where in the case of the R6/R7 ring closure this substituent may be optionally substituted singly by NH₂ or NO₂ or singly or doubly by OCH₃; and
R7 and R8 together cyclopentyl, diazirine or =CH₂;
where the compounds with R1 to R5 and R8 = H, n = 1 and R6/R7 = benzo-fused and R1, R3-R8 = H , R2 = CH₃ and n = 1 shall be excluded.

2. Benzotriazoles of the general formula I according to Claim 1,
in which the meanings are:
R1 to R8 H;
where one of these radicals R2 or R3 may represent:
R2 Br, Cl, CN, NO₂, CF₃, OCH₃, phenoxy, benzoyl, CH(OH)-phenyl, S-cyclohexyl, CO-OCH₃;
R3 CH₃, CN, Br, Cl, NH₂, NO₂, benzoyl.

3. Benzotriazoles of the general formula I according to Claim 1 or 2, in which the meanings are:
R1 to R8 H;
where one of these radicals R2 or R3 may represent:
R2 Br, Cl, NO₂, OCH₃, phenoxy, CO-OCH₃;
R3 NH₂; or
two substituents of this series are:
R2 = F and R3 = Cl;
n an integer from 1 or 2; and
one of the substituents R6 or R7 may represent:
R6 CH₃;
R7 CH₃, CF₃ or Br; or
the ring may contain a double bond instead of R6 and R7 or
R6 and R7 may together with the carbon atoms carrying them represent a benzo-fused ring which may optionally be substituted singly by NH₂ or singly or doubly by OCH₃; and
R7 and R8 together cyclopentyl or
n an integer from 0; and
R6 and R7 may together with the carbon atoms carrying them represent a benzo-fused ring or cyclohexanediyl.

4. Benzotriazoles of the general formula I as Claimed in claims 1 to 2, wherein
R1 to R8 H;
where one of these radicals R2 or R3 may represent:
R2 Br, CN, CF₃, OCH₃, phenoxy, benzoyl, CH(OH)-phenyl, S-cyclohexyl;
R3 CN, Br, Cl, NO₂, benzoyl; or
two substituents of this series are:
R1 = Cl and R3 = CF₃;
n an integer from 1; and
one of the substituents R6 and R7 may represent:
R6 CH₃ ;
R7 CH₃, C₂H₅; CH(CH₃)₂, C(CH₃)₃, benzyl or CO- OC₂H₅ ; or
R6 and R7 are both CH₃; or
the ring may contain a double bond instead of R6 and R7 or
R5 and R6 or R6 and R7 may together with the carbon atoms carrying them represent a benzo-fused ring;
where the compounds with R1 to R5 and R8 = H, n = 1 and R6/R7 = benzo-fused shall be excluded.

5. Benzotriazoles according to Claim 1 of the following structures:

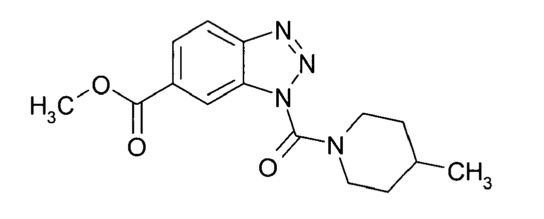

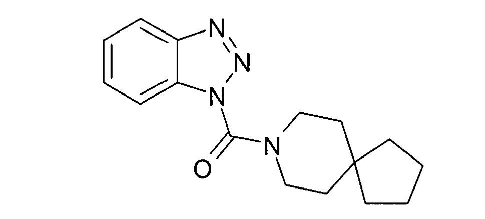

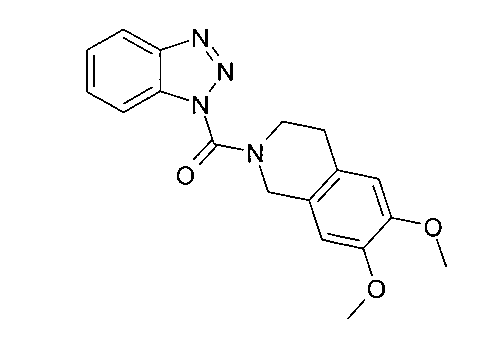

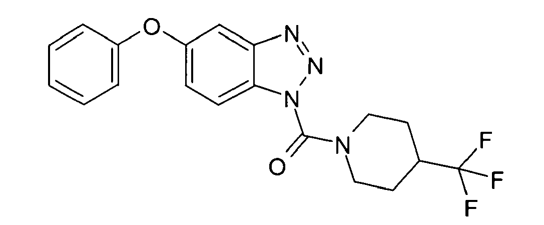

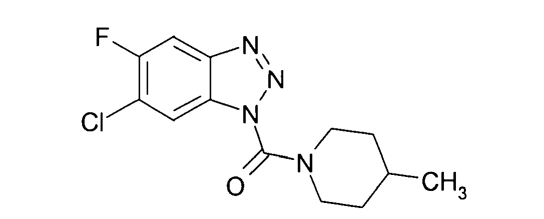

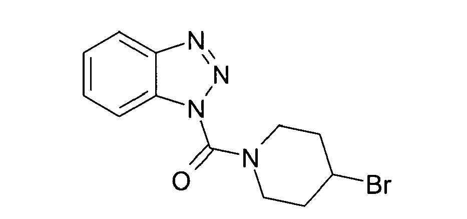

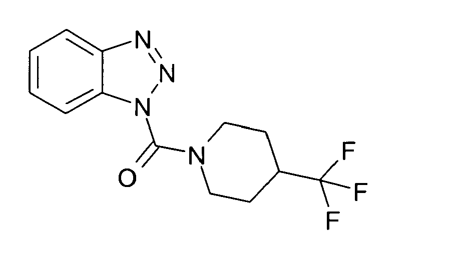

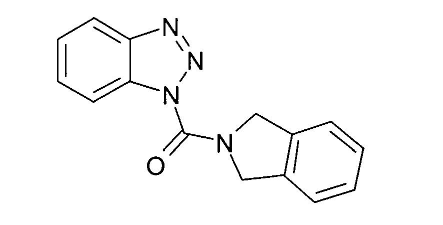

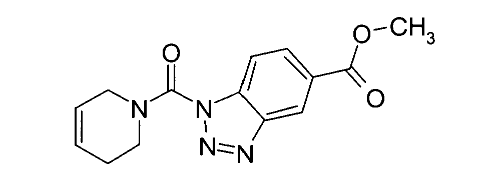

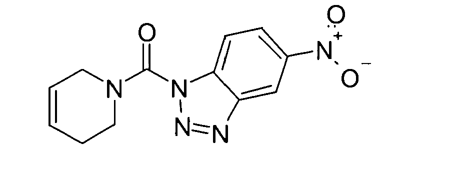

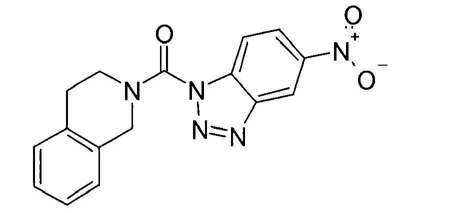

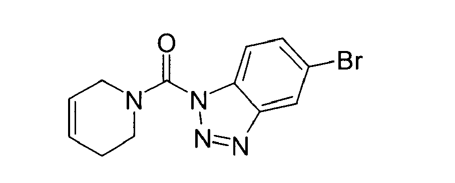

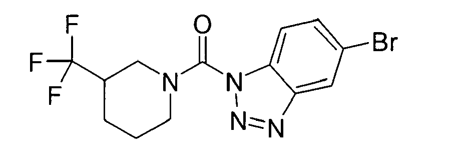

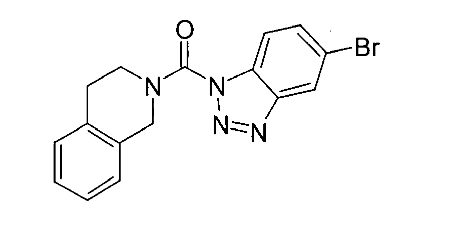

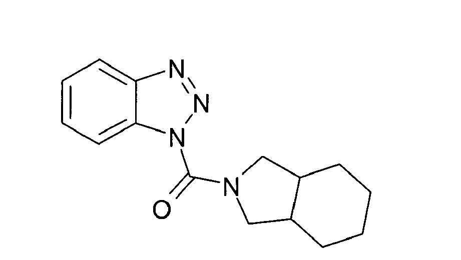

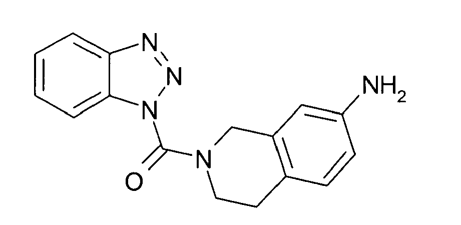

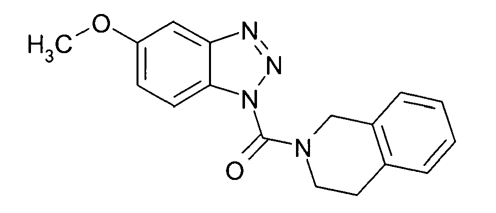

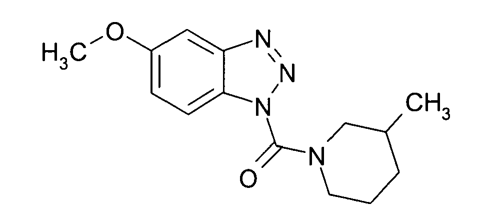

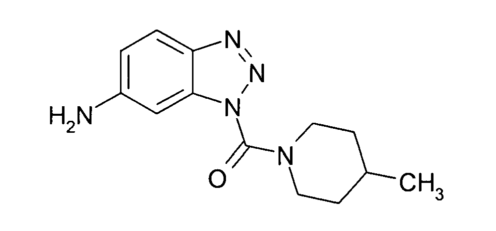

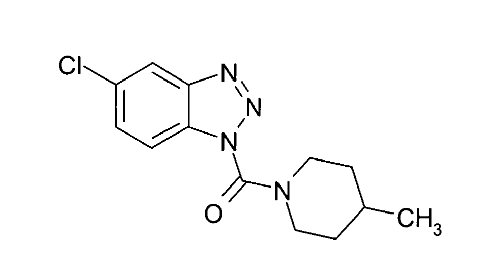

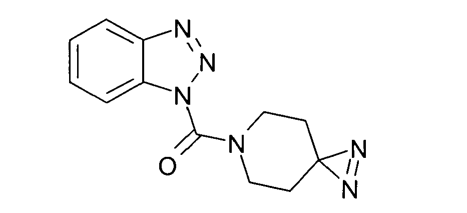

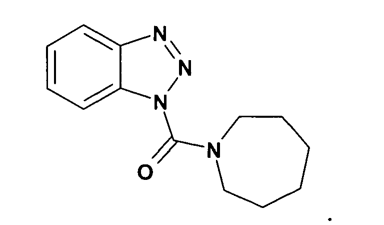

6. Benzotriazoles according to Claim 1 of the following structures:

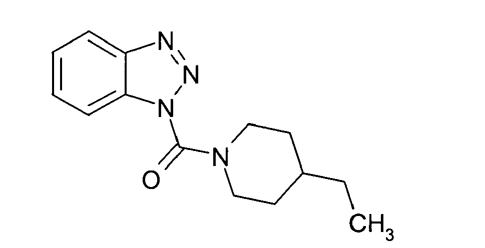

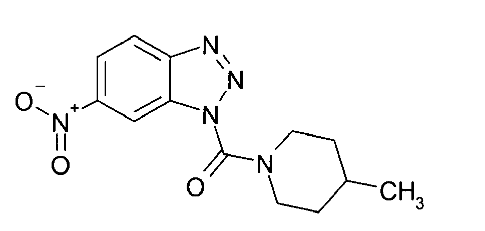

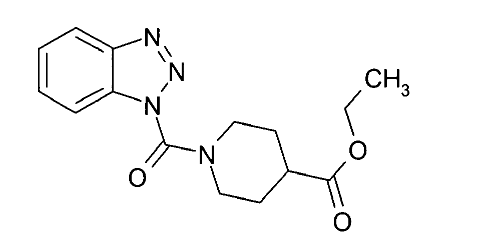

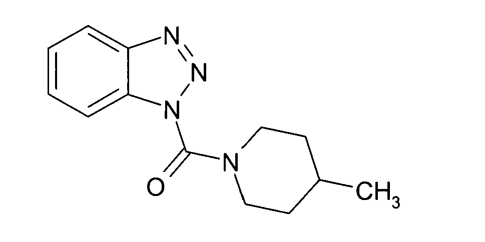

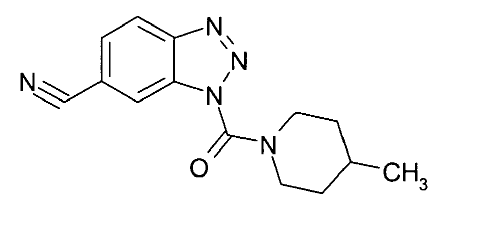

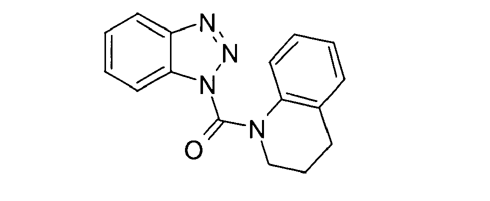

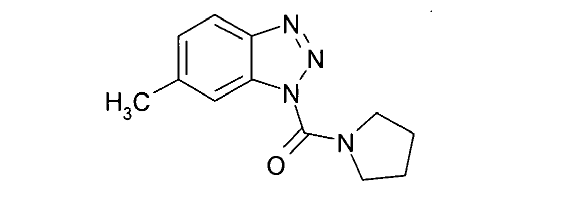

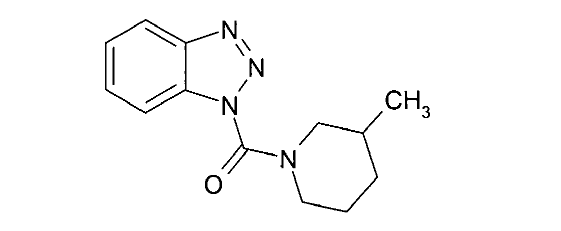

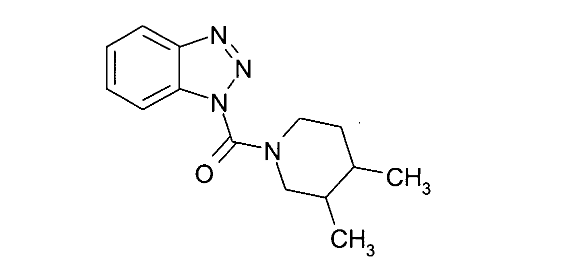

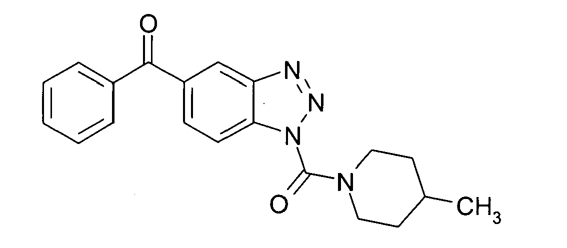

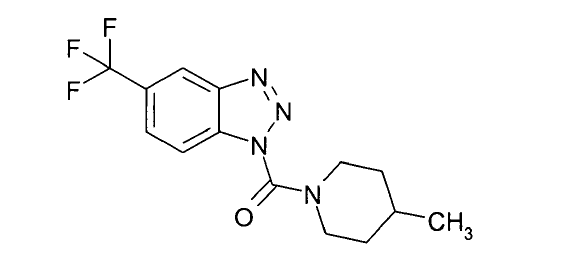

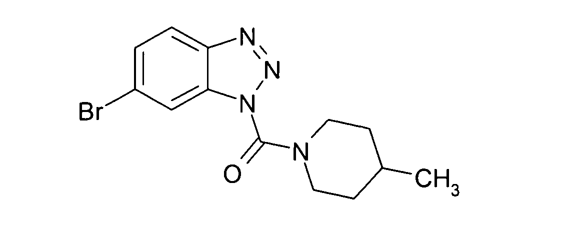

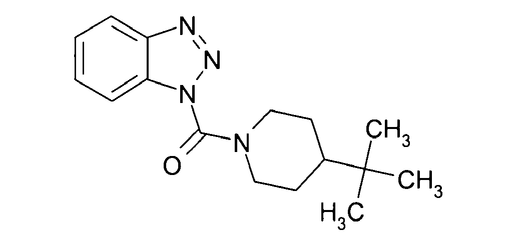

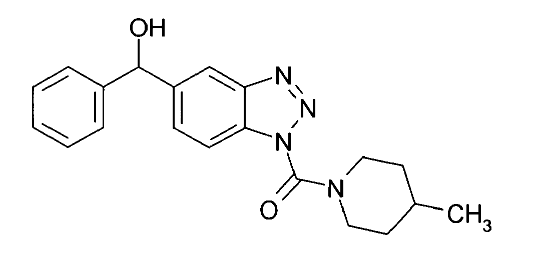

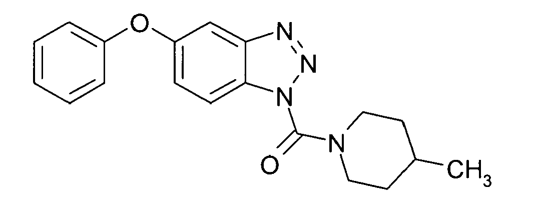

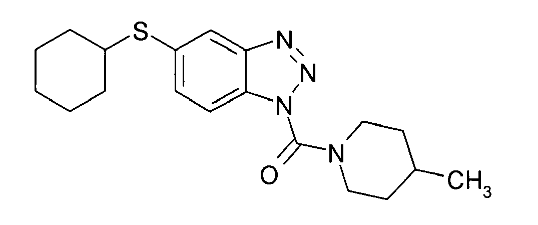

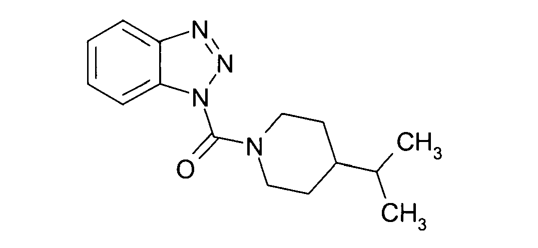

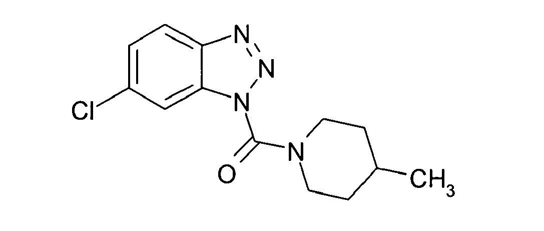

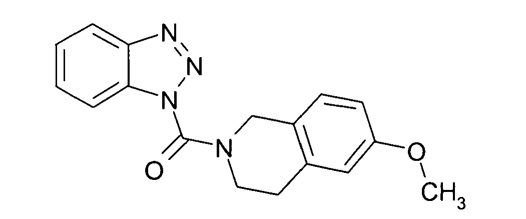

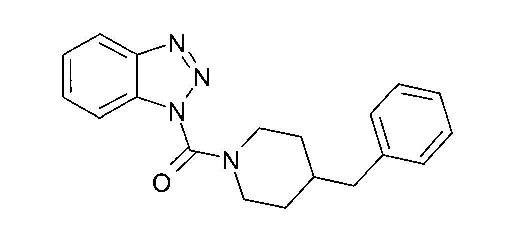

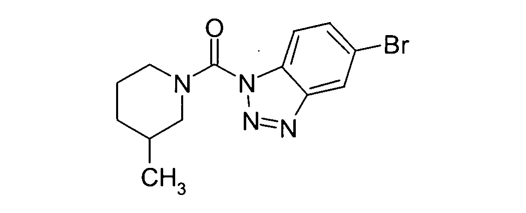

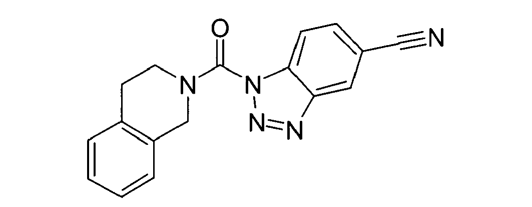

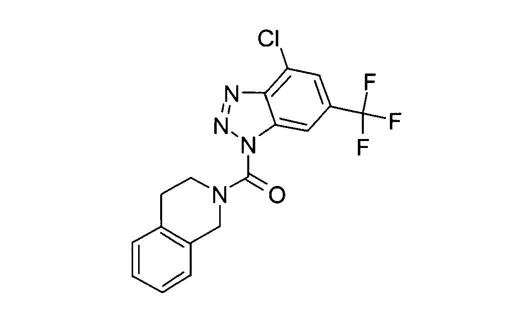

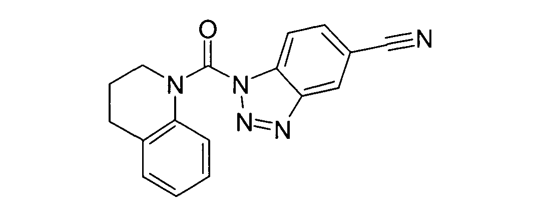

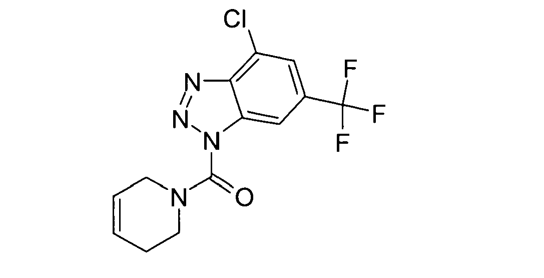

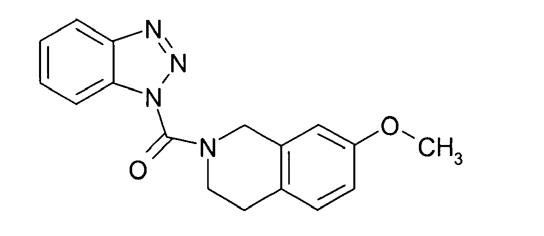

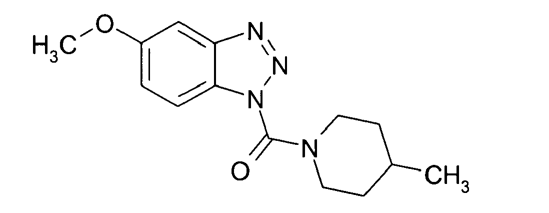

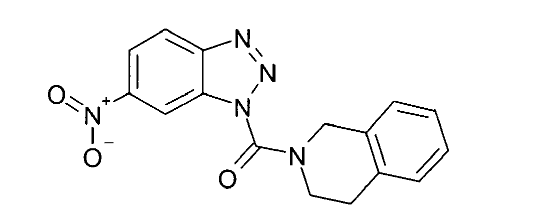

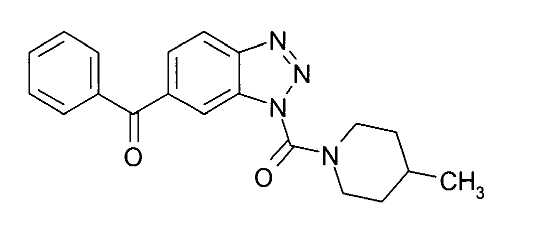

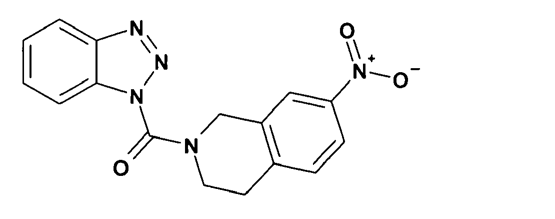

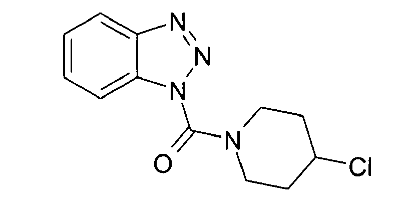

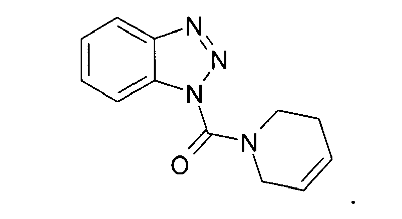

7. Benzotriazoles of the following structures as claimed in Claim 6:

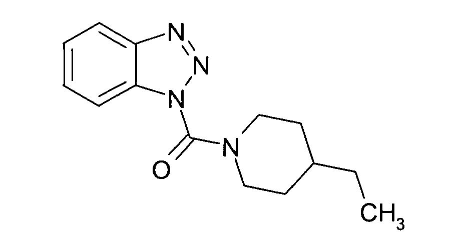

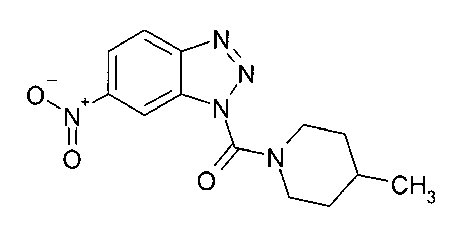

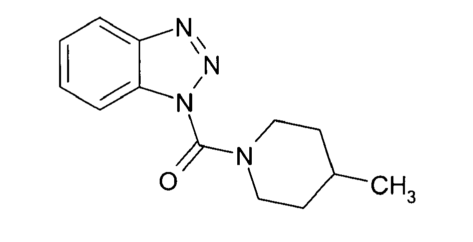

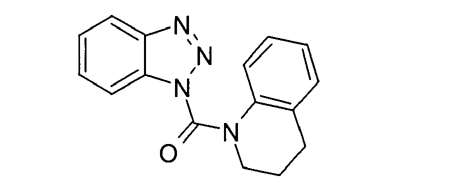

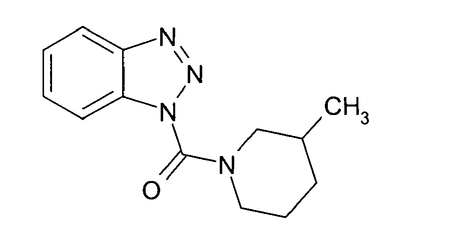

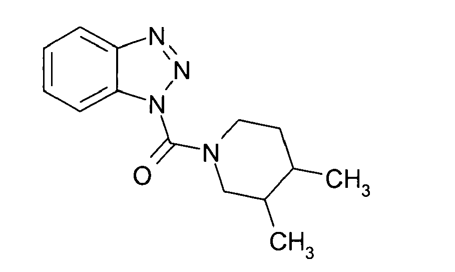

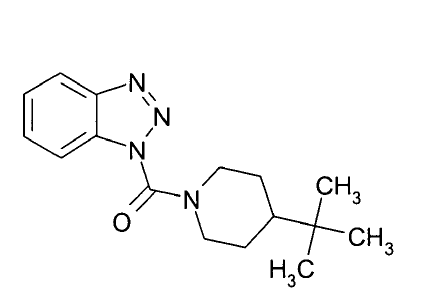

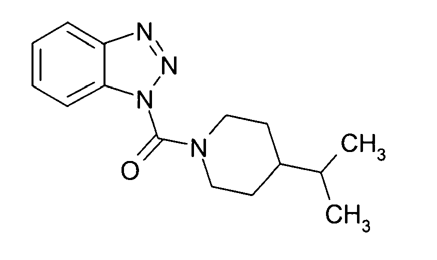

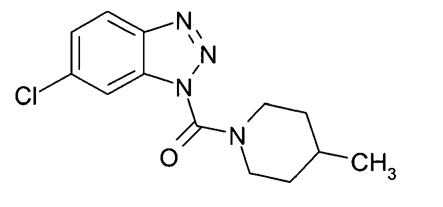

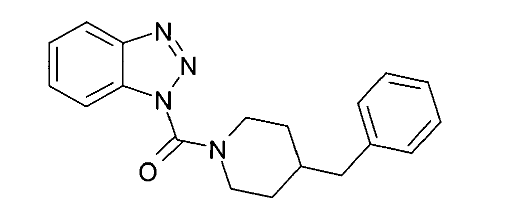

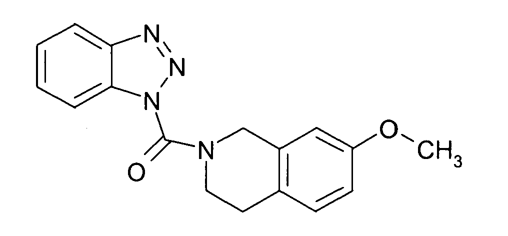

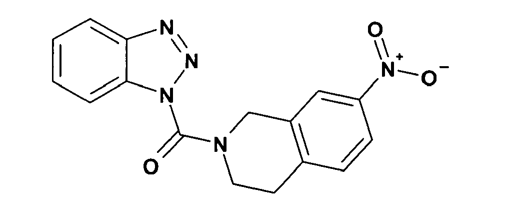

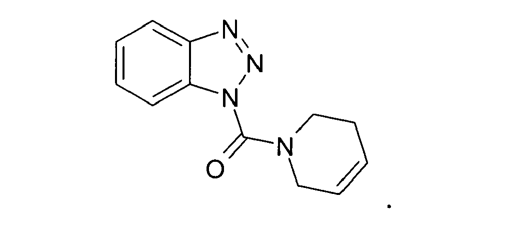

8. Benzotriazoles of the following structures as claimed in Claim 7:

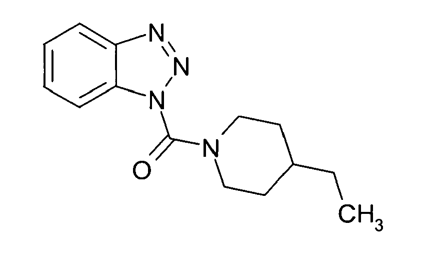

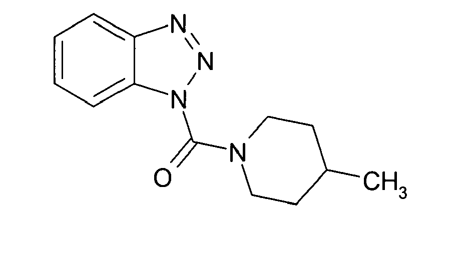

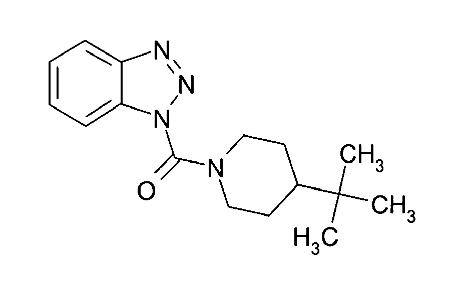

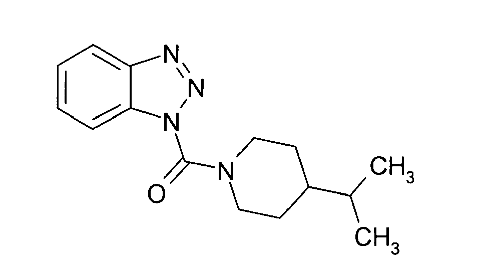

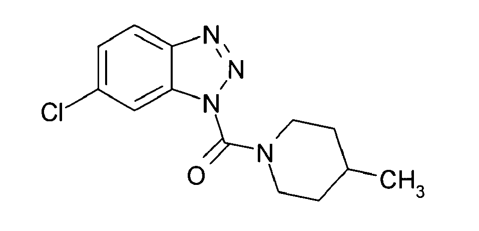

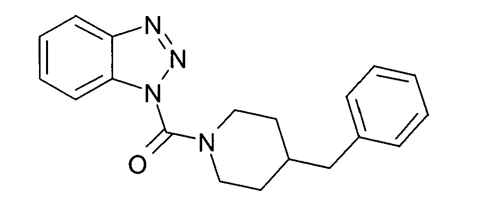

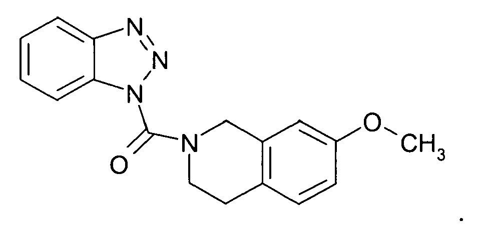

9. Benzotriazoles of the general formula I, 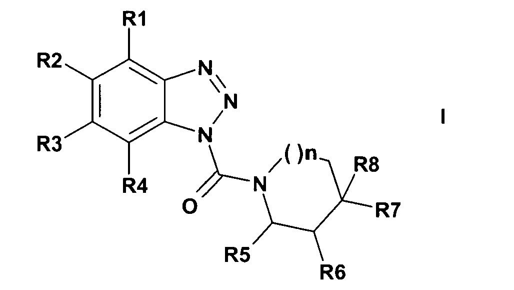 in which the meanings are:
R1 to R8 H;
where one of these radicals R2 or R3 may represent:
Br, Cl, CH₃, CN, NH₂, NO₂, CF₃, OCH₃, phenoxy, benzoyl, CH(OH)-phenyl, S-cyclohexyl, CO-OCH₃;
or
two substituents of this series are:
R1 = Cl and R3 = CF₃ or
R2 = F and R3 = Cl;
n an integer from 0, 1 or 2; and
one of the substituents R6 or R7 may represent:
R6 CH₃;
R7 CH₃, C₂H₅; CH(CH₃)₂, C(CH₃)₃, CF₃, Br, Cl, benzyl or CO-OC₂H₅; or
R6 and R7 are both CH₃; or
the ring may contain a double bond instead of R6 and R7 or
R5 and R6 or R6 and R7 may together with the carbon atoms carrying them represent a benzo-fused ring or else if n = 0 may represent cyclohexanediyl; where in the case of the R6/R7 ring closure this substituent may be optionally substituted singly by NH₂ or NO₂ or singly or doubly by OCH₃; and
R7 and R8 together cyclopentyl, diazirine or =CH₂;
for use in a medicament.

10. Benzotriazoles of the formula I as claimed in Claim 9 for use in a medicament with an inhibitory effect on hormone-sensitive lipase, HSL.

11. Benzotriazoles of the formula I as claimed in Claim 9 for use in a medicament for the treatment of non-insulin-dependent diabetes mellitus, of diabetic syndrome or syndrome X.

12. Medicament for the treatment of non-insulin-dependent diabetes mellitus or of diabetic syndrome comprising at least one benzotriazole of the formula I as claimed in Claim 9.

## Revendications

1. Benzotriazoles de formule générale I 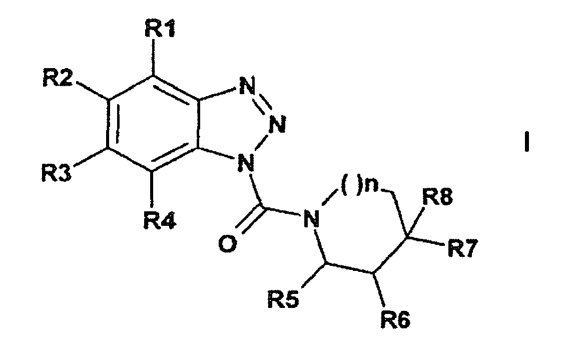 dans laquelle :
R1 à R8 représentent un atome d'hydrogène,
où l'un de ses radicaux R2 ou R3 peut représenter :
un atome de brome, un atome de chlore, un groupe CH₃, un groupe CN, un groupe NH₂, un groupe NO₂, un groupe CF₃, un groupe OCH₃, un groupe phénoxy, un groupe benzoyle, un groupe CH(OH)-phényle, un groupe S-cyclohexyle, un groupe CO-OCH₃ ;
ou
deux substituants de cette série sont :
R1 représentant un atome de chlore et
R3 représentant un groupe CF₃, ou
R2 représentant un atome de fluor et
R3 représentant un atome de chlore ;
n représentant un entier valant 0, 1 ou 2 ;
et
l'un des substituants R6 ou R7 peut représenter :
R6 un groupe CH₃,
R7 un groupe CH₃, un groupe C₂H₅, un groupe CH(CH₃)₂, un groupe C(CN₃)₃, un groupe CF₃, un atome de brome, un atome de chlore, un groupe benzyle ou un groupe CO-OC₂H₅ ; ou
R6 et R7 représentent tous les deux un groupe CH₃ ; ou
le noyau peut renfermer une double liaison au lieu de R6 et de R7 ; ou
R5 et R6, ou R6 et R7, conjointement avec les atomes de carbone les portant, peuvent représenter un noyau benzo-condensé ou si n vaut 0, peuvent également représenter un groupe cyclohexandiyle ; où dans le cas de la fermeture de cycles R6/R7, ce substituant peut éventuellement être mono-substitué par un groupe NH₂ ou un groupe NO₂, ou mono- à bi-substitué par un groupe OCH₃ ;
et
R7 et R8 représentent conjointement un groupe cyclopentyle, un groupe diazirine ou un groupe =CH₂ ;
où les composés, dans lesquels R1 à R5 et R8 représentent un atome d'hydrogène, n vaut 1 et R6/R7 est benzo-condensé, et R1, R3 à R8 représentent un atome d'hydrogène, R2 représente un groupe CH₃ et n vaut 1, sont exclus.

2. Benzotriazoles de formule générale I selon la revendication 1, dans laquelle :
R1 à R8 représentent un atome d'hydrogène,
où l'un de ses radicaux R2 ou R3 peut représenter :
R2 un atome de brome, un atome de chlore, un groupe CN, un groupe NO₂, un groupe CF₃, un groupe OCH₃, un groupe phénoxy, un groupe benzoyle, un groupe CH(OH)-phényle, un groupe S-cyclohexyle, un groupe CO-OCH₃ ;
R3 un groupe CH₃, un groupe CN, un atome de brome, un atome de chlore, un groupe NH₂, un groupe NO₂, un groupe benzoyle.

3. Benzotriazoles de formule générale I selon la revendication 1 ou 2, dans laquelle :
R1 à R8 représentent un atome d'hydrogène,
où l'un de ses radicaux R2 ou R3 peut représenter :
R2 un atome de brome, un atome de chlore, un groupe NO₂, un groupe OCH₃, un groupe phénoxy, un groupe CO-OCH₃ ;
R3 un groupe NH₂ ; ou
deux substituants de cette série sont :
R2 représentant un atome de fluor et
R3 représentant un atome de chlore ;
n représentant un entier valant 1 ou 2 ; et
l'un des substituants R6 ou R7 peut représenter :
R6 un groupe CH₃,
R7 un groupe CH₃, un groupe CF₃ ou un atome de brome ; ou
le noyau peut renfermer une double liaison au lieu de R6 et de R7 ; ou
R6 et R7, conjointement avec les atomes de carbone les portant, peuvent représenter un noyau benzo-condensé, qui peut être éventuellement mono-substitué par un groupe NH₂, ou mono- à bi-substitué par un groupe OCH₃ ; et
R7 et R8 représentent conjointement un groupe cyclopentyle, ou
n représente un entier valant 0 ; et
R6 et R7, conjointement avec les atomes de carbone les portant, peuvent représenter un noyau benzo-condensé ou un groupe cyclohexandiyle.

4. Benzotriazoles de formule générale I selon les revendications 1 à 2, dans laquelle :
R1 à R8 représentent un atome d'hydrogène,
où l'un de ses radicaux R2 ou R3 peut représenter :
R2 un atome de brome, un groupe CN, un groupe CF₃, un groupe OCH₃, un groupe phénoxy, un groupe benzoyle, un groupe CH(OH)-phényle, un groupe S-cyclohexyle ;
R3 un groupe CN, un atome de brome, un atome de chlore, un groupe NO₂, un groupe benzoyle ; ou
deux substituants de cette série sont :
R1 représentant un atome de chlore et
R3 représentant un groupe CF₃ ;
n représentant un entier valant 1 ; et
l'un des substituants R6 ou R7 peut représenter :
R6 un groupe CH₃,
R7 un groupe CH₃, un groupe C₂H₅, un groupe CH(CH₃)₂, un groupe C(CH₃)₃, un groupe benzyle ou un groupe CO-OC₂H₅ ; ou
R6 et R7 représentent tous les deux un groupe CH₃ ; ou
le noyau peut renfermer une double liaison au lieu de R6 et de R7 ; ou
R5 et R6, ou R6 et R7, conjointement avec les atomes de carbone les portant, peuvent représenter un noyau benzo-condensé ;
où les composés, dans lesquels R1 à R5 et R8 représentent un atome d'hydrogène, n vaut 1 et R6/R7 est benzo-condensé, sont exclus.

5. Benzotriazoles selon la revendication 1 de structures suivantes :

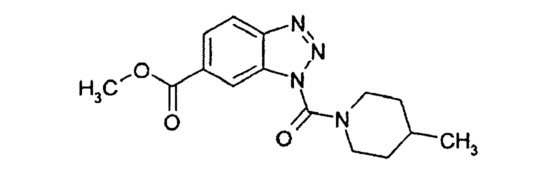

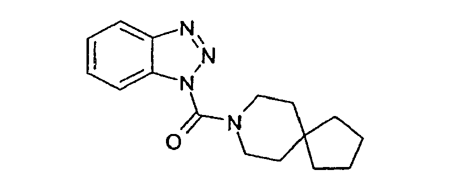

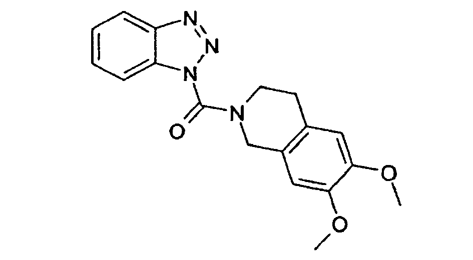

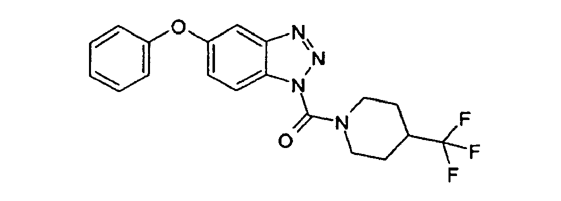

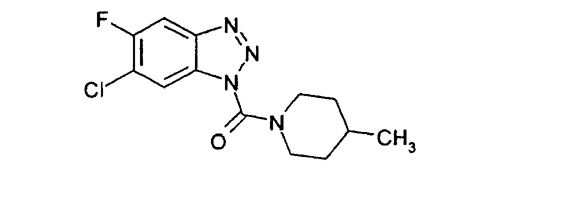

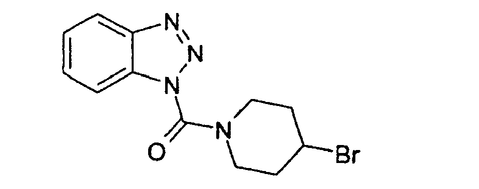

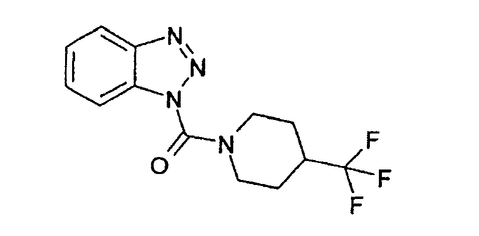

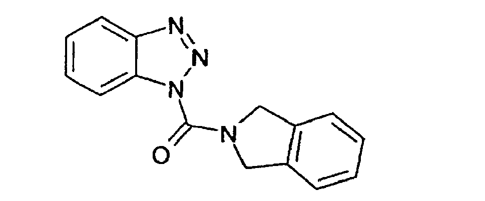

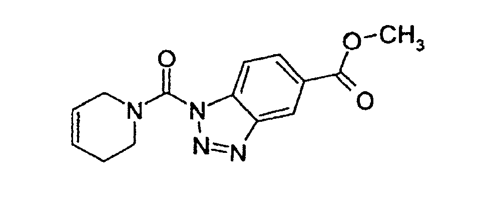

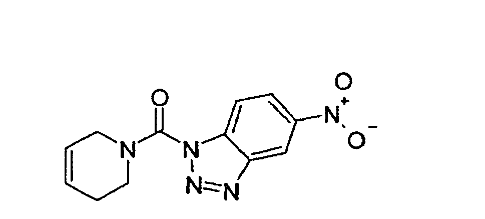

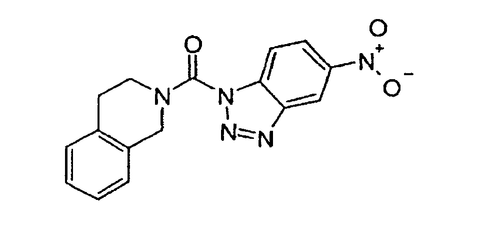

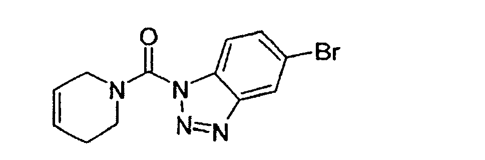

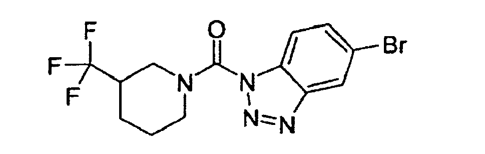

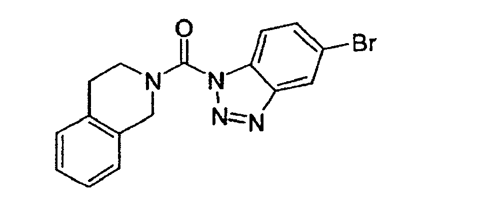

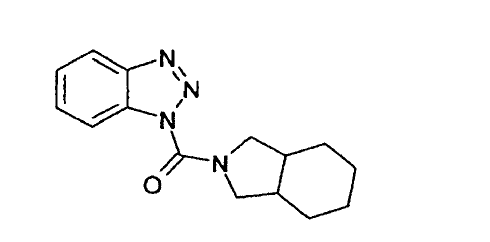

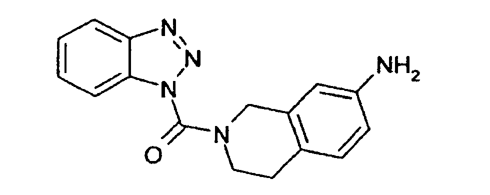

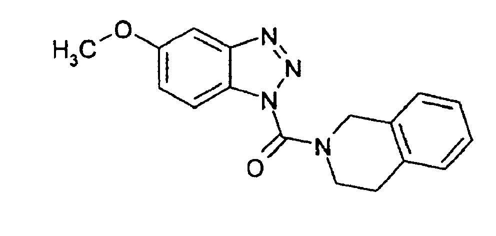

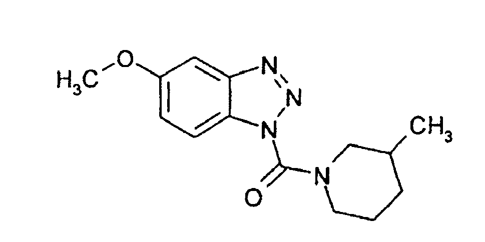

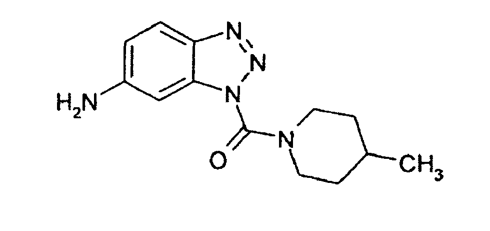

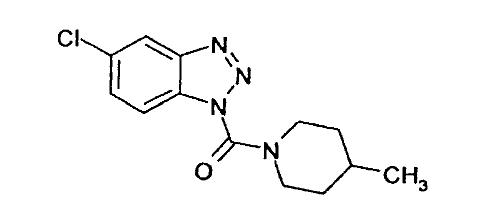

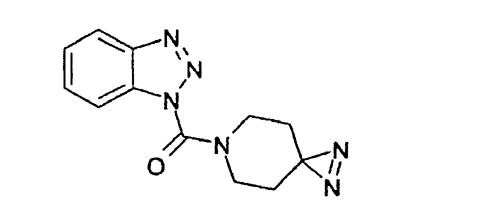

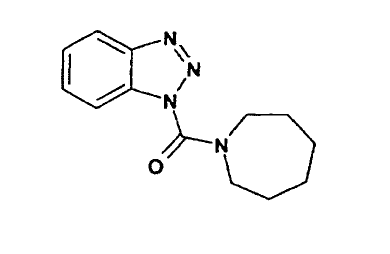

6. Benzotriazoles selon la revendication 1 de structures suivantes :

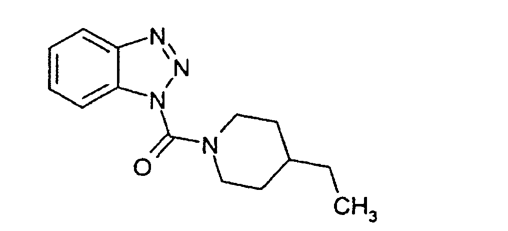

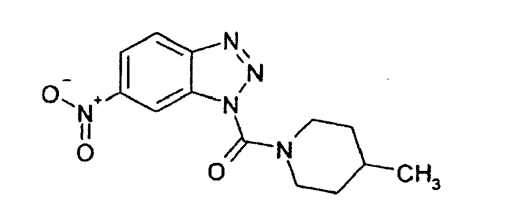

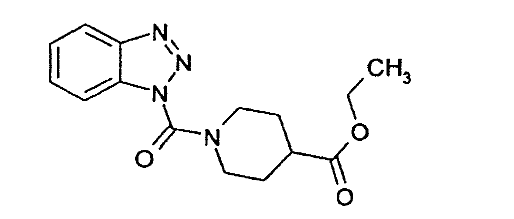

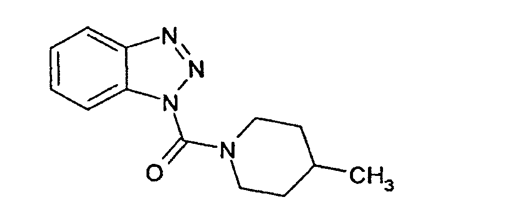

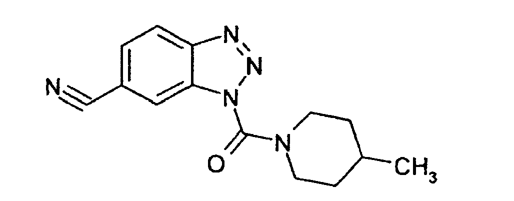

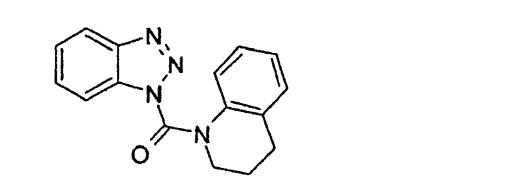

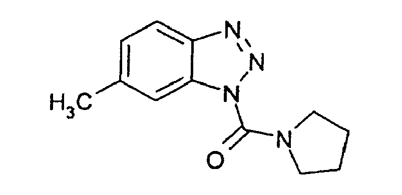

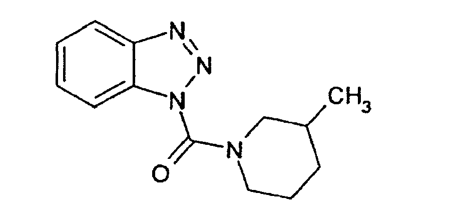

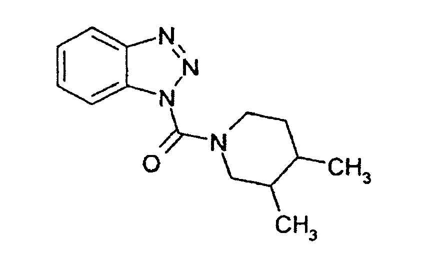

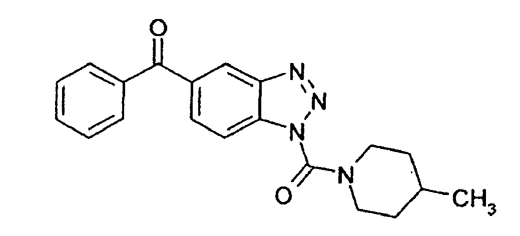

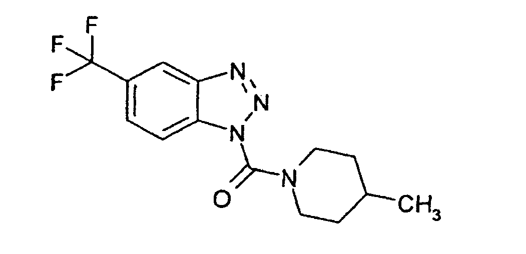

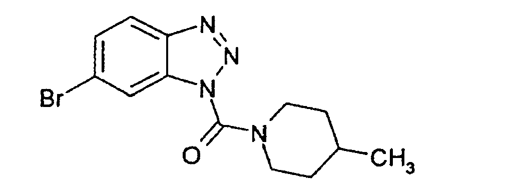

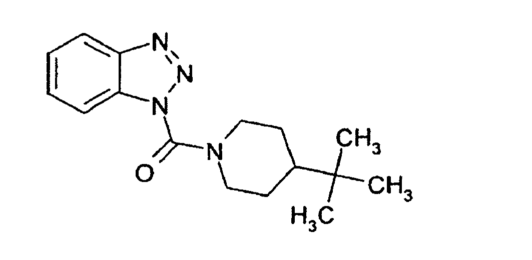

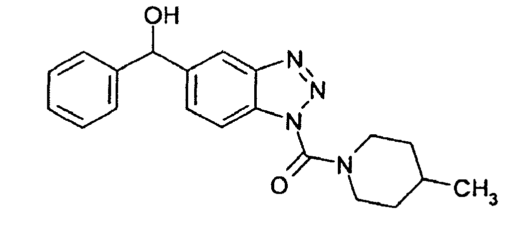

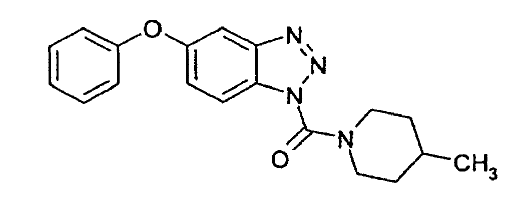

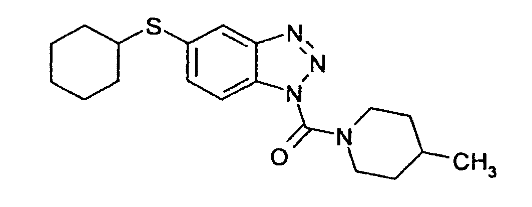

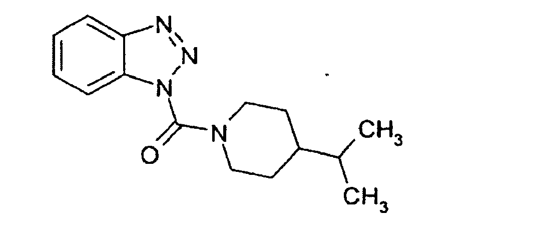

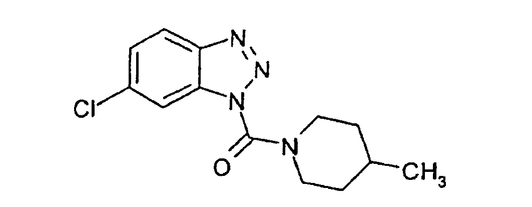

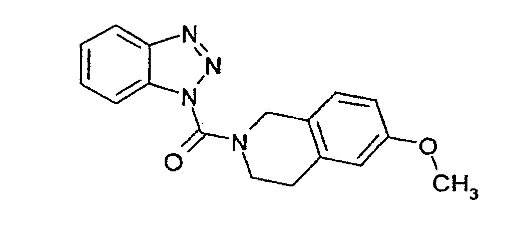

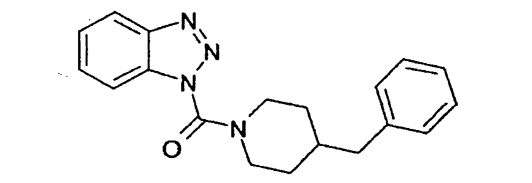

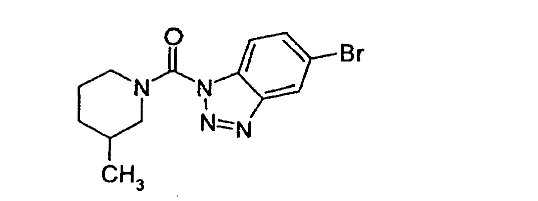

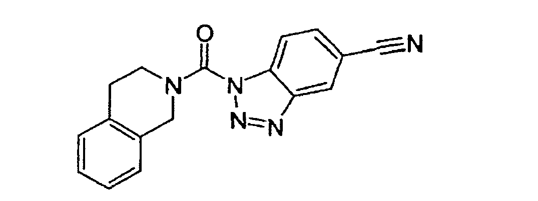

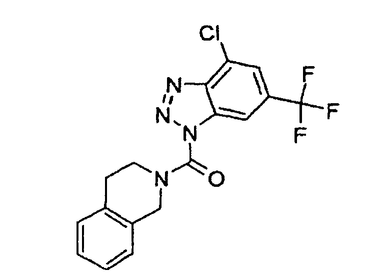

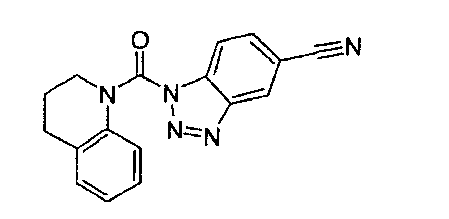

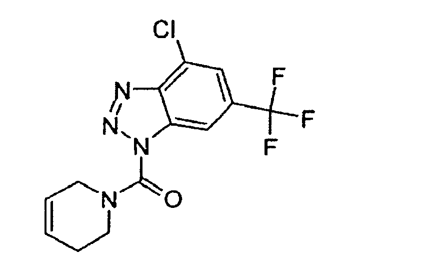

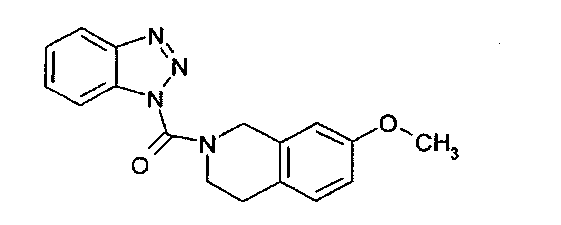

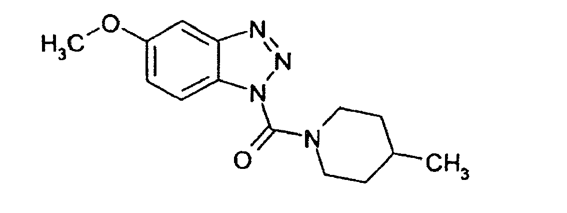

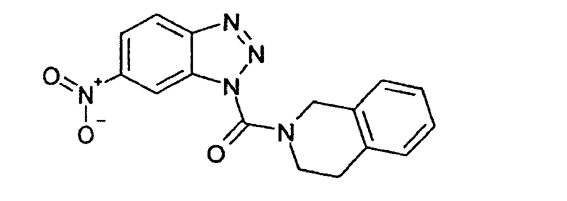

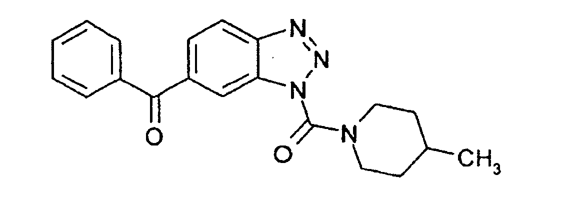

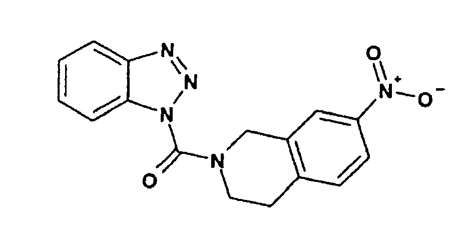

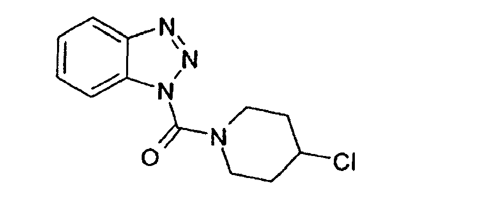

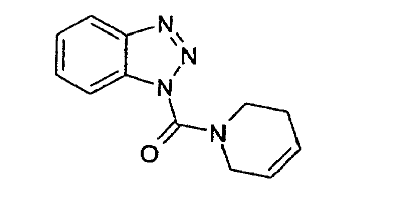

7. Benzotriazoles de structures suivantes selon la revendication 6 :

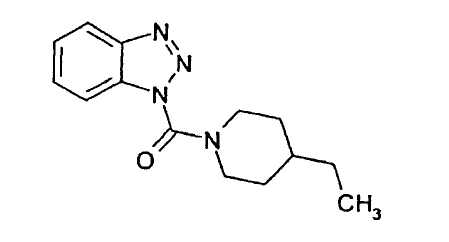

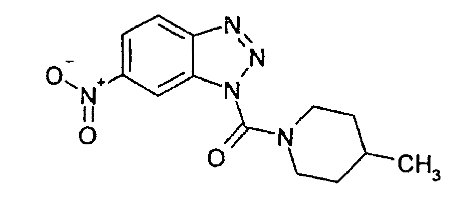

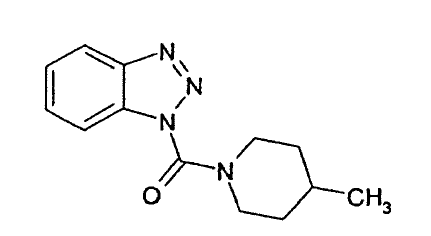

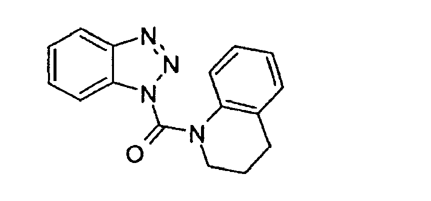

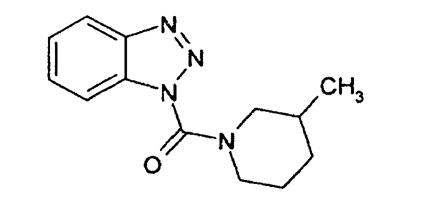

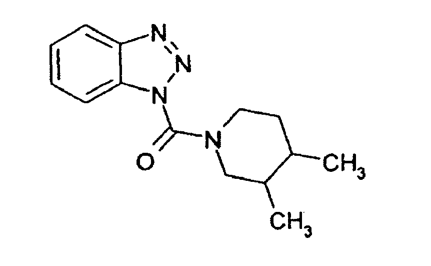

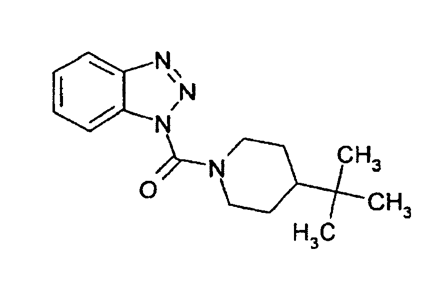

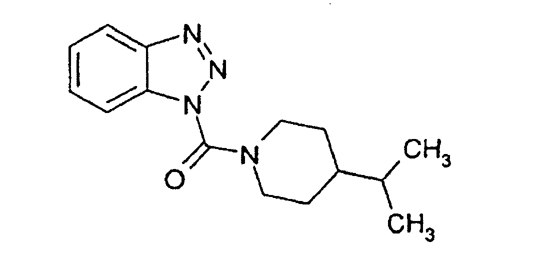

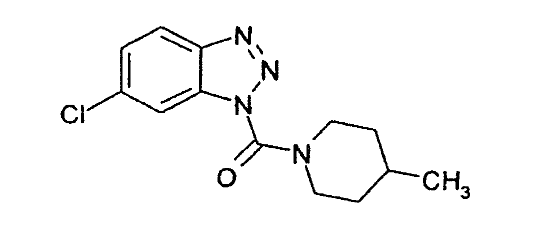

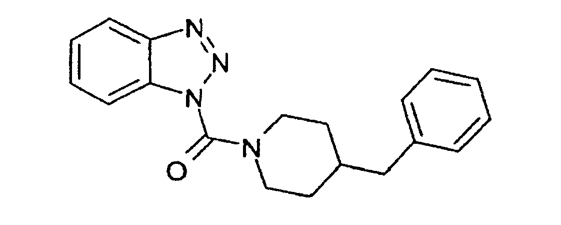

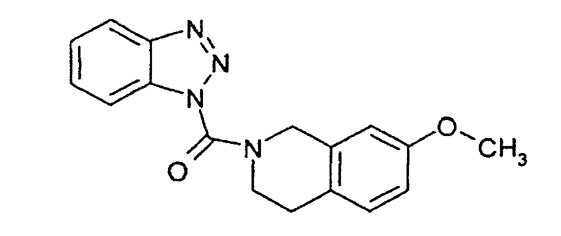

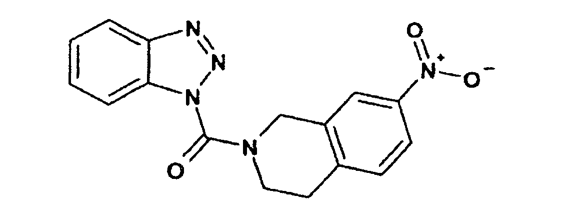

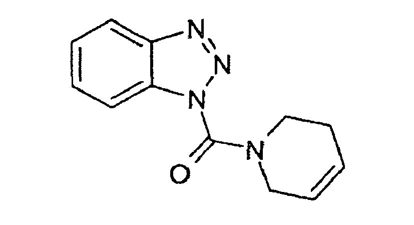

8. Benzotriazoles de structures suivantes selon la revendication 7 :

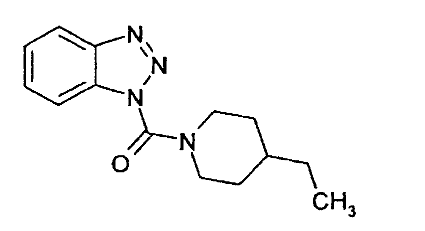

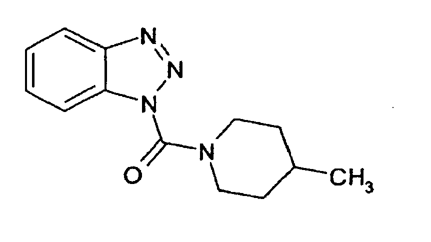

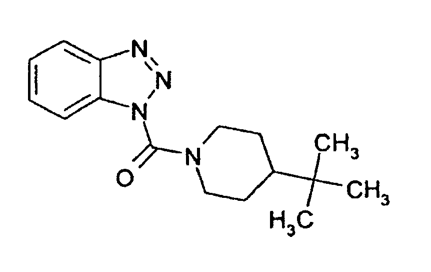

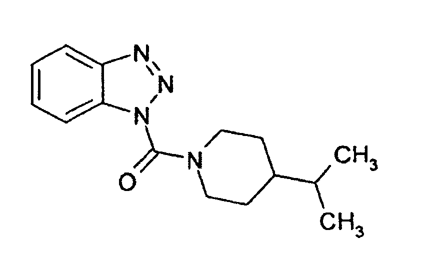

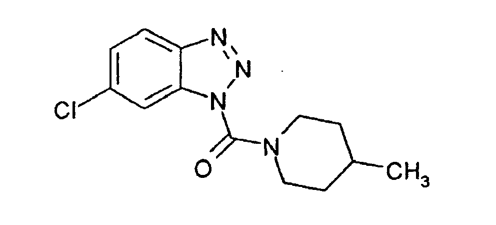

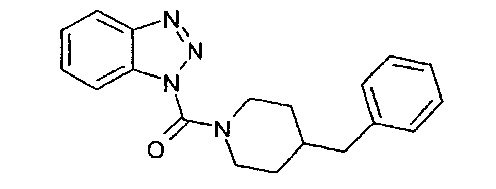

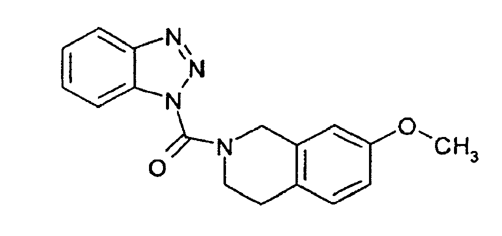

9. Benzotriazoles de formule générale I 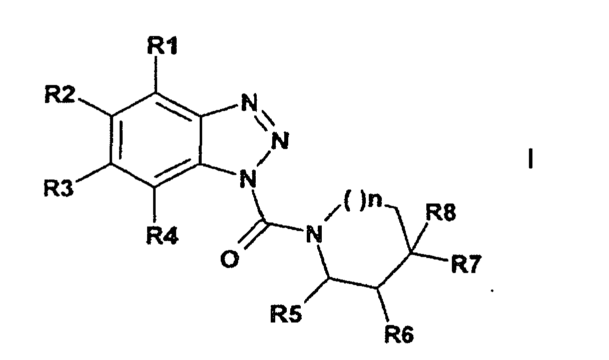 dans laquelle :
R1 à R8 représentent un atome d'hydrogène,
où l'un de ses radicaux R2 ou R3 peut représenter :
un atome de brome, un atome de chlore, un groupe CH₃, un groupe CN, un groupe NH₂, un groupe NO₂, un groupe CF₃, un groupe OCH₃, un groupe phénoxy, un groupe benzoyle, un groupe CH(OH)-phényle, un groupe S-cyclohexyle, un groupe CO-OCH₃ ;
ou
deux substituants de cette série sont :
R1 représentant un atome de chlore et
R3 représentant un groupe CF₃, ou
R2 représentant un atome de fluor et
R3 représentant un atome de chlore ;
n représentant un entier valant 0, 1 ou 2 ;
et
l'un des substituants R6 ou R7 peut représenter :
R6 un groupe CH₃,
R7 un groupe CH₃, un groupe C₂H₅, un groupe CH(CH₃)₂, un groupe C(CH₃)₃, un groupe CF₃, un atome de brome, un atome de chlore, un groupe benzyle ou un groupe CO-OC₂H₅ ; ou
R6 et R7 représentent tous les deux un groupe CH₃ ; ou
le noyau peut renfermer une double liaison au lieu de R6 et de R7 ; ou
R5 et R6, ou R6 et R7, conjointement avec les atomes de carbone les portant, peuvent représenter un noyau benzo-condensé ou si n vaut 0, peuvent également représenter un groupe cyclohexandiyle ; où dans le cas de la fermeture de cycles R6/R7, ce substituant peut éventuellement être mono-substitué par un groupe NH₂ ou un groupe NO₂, ou mono- à bi-substitué par un groupe OCH₃ ;
et
R7 et R8 représentent conjointement un groupe cyclopentyle, un groupe diazirine ou un groupe =CH₂ ;
pour une utilisation dans un médicament.

10. Benzotriazoles de formule I selon la revendication 9 pour une utilisation dans un médicament présentant une action inhibitrice sur la lipase hormono-sensible HSL.

11. Benzotriazoles de formule I selon la revendication 9 pour une utilisation dans un médicament destiné au traitement du diabète sucré non insulino-dépendant, du syndrome diabétique ou du syndrome X.

12. Médicament destiné au traitement du diabète sucré non insulino-dépendant ou du syndrome diabétique, comprenant au moins un benzotriazole de formule I selon la revendication 9.
